# EUROPEAN PATENT APPLICATION

(11) **EP 1 878 733 A1**
(43) Date of publication of application: **16.01.2008**
(21) Application number: 06117227.6
(22) Date of filing: 14.07.2006
(51) Int. Cl.: C07D 401/14, C07D 405/14, C07D 413/14, C07D 471/04, C07D 417/14, C07D 409/14, A61K 31/435, A61P 9/00, A61P 11/00, A61P 29/00

(54) **Pyrimidine derivatives as ALK-5 inhibitors**

(71) Applicant: Novartis AG, 4056 Basel (CH)
(72) Inventor: The designation of the inventor has not yet been filed
(74) Representative: McLean, Craig Sutherland

(57) **Abstract**

Compounds of formula I 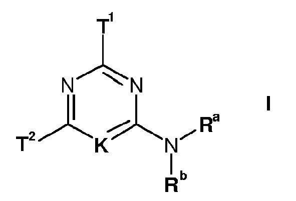 in free or salt or solvate form, where T¹, T², K, R^{a} and R^{b} have the meanings as indicated in the specification, are useful for treating diseases mediated by the ALK-5 and/or ALK-4 receptor. Pharmaceutical compositions that contain the compounds and processes for preparing the compounds are also described.

## Description

This invention relates to organic compounds and their use as pharmaceuticals, in particular for the treatment of inflammatory or obstructive airways diseases such as pulmonary hypertension and systemic skeletal disorders such as osteoporosis.

In one aspect, the present invention provides a compound of formula I 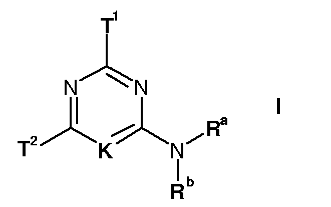 in free or salt or solvate form, where
T¹ is a 4- to 14-membered heterocyclic group optionally substituted at one, two or three positions by R¹, C₁-C₈-alkoxy, C₁-C₈-alkylthio, halo, halo-C₁-C₈-alkyl, amino, C₁-C₈-alkylamino, di(C₁-C₈-alkyl)amino, cyano, oxo, hydroxy, carboxy, nitro or by C₃-C₁₀-cycloalkyl optionally substituted at one, two or three positions by hydroxy, cyano, amino or R¹;
T² is a 4- to 14-membered heterocyclic group optionally substituted at one, two or three positions by R¹, R², R⁵, C₁-C₈-alkoxy, C₁-C₈-alkylthio, halo, halo-C₁-C₈-alkyl, amino, -C₁-C₈-alkylamino, di(C₁-C₈-alkyl)amino, cyano, oxo, hydroxy, carboxy, nitro or by C₃-C₁₀-cycloalkyl optionally substituted at one, two or three positions by hydroxy, cyano, amino or R¹;
K is -CR³ or N ;
either R^{a} and R^{b} are independently hydrogen;
   C₁-C₈-alkyl optionally substituted at one, two or three positions by R⁴;
   C₃-C₁₀-cycloalkyl optionally substituted at one, two or three positions by hydroxy, amino, C₁-C₈-alkoxy, C₁-C₈-alkylthio, halo, halo-C₁-C₈-alkyl, C₁-C₈-alkylamino, di(C₁-C₈-alkyl)amino, cyano, oxo, carboxy, nitro, -R¹, R², R⁵, -C(=O)-C₁-C₈-alkyl-R⁵,
      -C(=O)-C₁-C₈-alkyl-R², -C(=O)-NR⁶R⁷, -SO₂NR⁶R⁷, -SO₂-R² or -SO₂-R⁵, where C₃-C₁₀-cycloalkyl is optionally fused to a phenyl group;
   -C(=O)-C₁-C₈-alkyl-R⁵, -C(=O)-R⁵, -C(=O)-C₁-C₈-alkyl-R², -C(=O)- R², -C (=O)-NR⁶R⁷, -SO₂NR⁶R⁷, -SO₂-R² or SO₂-R⁵;
   a 4- to 14-membered heterocyclic group optionally substituted at one, two or three positions by hydroxy, C₁-C₈-alkoxy, C₁-C₈-alkylthio, halo, halo-C₁-C₈-alkyl, amino, C₁-C₈-alkylamino, di(C₁-C₈-alkyl)amino, cyano, oxo, carboxy, nitro, -R¹, -R², -R⁵ or -C(=O)-O-R¹; or
   C₆-C₁₅-aryl optionally substituted at one, two or three positions by halo, hydroxy, C₁-C₈-alkoxy, C₁-C₈-alkylthio, halo-C₁-C₈-alkyl, amino, C₁-C₈-alkylamino, di(C₁-C₈-alkyl)amino, cyano, oxo, carboxy, nitro, -R⁵, -SO₂-R⁵ or -C(=O)-O-R¹;
or R^{a} and R^{b} together form a 4- to 14-membered heterocyclic group optionally substituted at one, two or three positions by oxo, -R¹, C₁-C₈-alkoxy, C₁-C₈-alkylthio, halo, amino, C₁-C₈-alkylamino, di(C₁-C₈-alkyl)amino, cyano, hydroxy, carboxy, -SO₂-R¹, -SO₂-R², -SO₂-R⁵, -SO₂NR⁶R⁷ or nitro;
R¹ is C₁-C₈-alkyl, C₂-C₈-alkenyl or C₂-C₈-alkynyl optionally substituted at one, two or three positions by hydroxy, cyano, amino, halo or -O-C₁-C₈-alkyl;
R² is C₆-C₁₅-aryl optionally substituted at one, two or three positions by halo, hydroxy, -R¹, -R⁵, C₁-C₈-alkoxy, C₁-C₈-alkylthio, amino, C₁-C₈-alkylamino, di(C₁-C₈-alkyl)amino, cyano, carboxy, nitro, -O-C₆-C₁₅-aryl, halo-C₁-C₈-alkyl, -NR⁶R⁷, -C₁-C₈-alkyl-NR⁶R⁷, -O-C₁-C₈-alkyl-NR⁶R4⁷, -C₁-C₈-alkyl-R⁵, -O-R¹, -O-R⁵, -C(=O)-NH₂, -C(=O)NR⁶R⁷, -C(=O)-O-R¹, -O-C(=O)-R¹, -SO₂-NH₂, -SO₂-R¹, -SO₂-C₆-C₁₅-aryl, -SO₂-R⁵ or-SO₂NR⁶R⁷;
R³ is hydrogen, halo, cyano, amino or -R¹;
R⁴ is hydroxy, amino, C₁-C₈-alkoxy, C₁-C₈-alkylthio, halo, halo-C₁-C₈-alkyl, C₁-C₈-alkylamino, di(C₁-C₈-alkyl)amino, cyano, carboxy, nitro, -N(H)-C(=NH)-NH₂, -N(H)-SO₂-R², -R², -C(=O)-R², -C(=O)-R⁵, -O-R², -O-R⁵, -N(H)-R⁵, -N(H)-R², - NR⁶R⁷, -C(=O)-R¹, -C(=O)-NH₂, -SO₂-R⁵, -C(=O)-O-R¹, -C(=O)-O-R², -C(=O)-O-R⁵, -SO₂-R² or -C(=O)-N(H)-C₁-C₈-alkyl-C(=O)-N(H)-R²;
   or R⁴ is a 4- to 14-membered heterocyclic group, that group being optionally substituted at one, two or three positions by hydroxy, halo, oxo, cyano, -R¹, amino, C₁-C₈-alkylamino, di(C₁-C₈-alkyl)amino, carboxy, nitro, -N(H)R¹, -C(=O)-NH₂, -C(=O)-NR⁶R⁷, -C(=O)-R², -C(=O)-R⁵ , C₁-C₈-alkoxy, C₁-C₈-alkylthio, C₃-C₁₀-cycloalkyl, -C(=O)-R¹, halo-C₁-C₈-alkyl, -R², -C₁-C₈-alkyl-R², -R⁵, -SO₂-R¹, -SO₂-R^{2,} , -SO₂-R⁵ or -SO₂NR⁶R⁷;
   or R⁴ is C₃-C₁₀-cycloalkyl substituted at one, two or three positions by hydroxy, amino, halo, C₁-C₈-alkylamino, di(C₁-C₈-alkyl)amino, cyano, carboxy, nitro -R¹, halo-C₁-C₈-alkyl, R², R⁵,
   -C(=O)-C₁-C₈-alkyl-R⁵, -C(=O)-C₁-C₈-alkyl-R², -C(=O)-NR⁶R⁷, -SO₂NR⁶R⁷, -SO₂-R², - SO₂-R⁵,
   -C(=O)-NH₂,, -SO₂-NH₂ or -SO₂-R¹;
R⁵ is a 4- to 14-membered heterocyclic group optionally substituted at one, two or three positions by oxo, amino, halo, C₁-C₈-alkylamino, di(C₁-C₈-alkyl)amino, cyano, hydroxy, carboxy, nitro, -R¹, C₁-C₈-alkoxy, C₁-C₈-alkylthio, halo-C₁-C₈-alkyl, -C(=O)-NH₂, -SO₂-NH₂,
   -SO₂-C₆-C₁₅-aryl or by a 4- to 14-membered heterocyclic group optionally substituted at one, two or three positions by -R¹; and
R⁶ and R⁷ are independently hydrogen, -R¹, C₁-C₈-alkylamino, di(C₁-C₈-alkyl)amino, C₆-C₁₅-aryl, -C₁-C₈-alkyl-C₆-C₁₅-aryl, -R⁵ or -C₁-C₈-alkyl-R⁵.

Terms used in the specification have the following meanings:
" Optionally substituted at one, two or three positions" as used herein means the group referred to can be substituted at one or two or three positions by any one or any combination of the radicals listed thereafter.
"Halo" or "halogen" as used herein denotes a element belonging to group 17 (formerly group VII) of the Periodic Table of Elements, which may be, for example, fluorine, chlorine, bromine or iodine.
"C₁-C₈-alkyl" as used herein denotes straight chain or branched alkyl that contains one to eight carbon atoms.
"C₂-C₈-alkenyl" as used herein denotes straight chain or branched hydrocarbon chains that contain two to ten carbon atoms and one or more carbon-carbon double bonds. "C₂-C₈-alkynyl" as used herein denotes straight chain or branched hydrocarbon chains that contain two to eight carbon atoms and one or more carbon-carbon triple bonds.
"C₆-C₁₅-aryl", as used herein, denotes an aromatic group having 6- to 15-ring carbon atoms. Examples of C₆-C₁₅-aryl groups include but are not limited to phenyl, phenylene, benzenetriyl, indanyl, naphthyl, naphthylene, naphthalenetriyl and anthrylene,
"4- to 14-membered heterocyclic group", refers to a 4- to 14-membered heterocyclic ring containing at least one ring heteroatom selected from the group consisting of nitrogen, oxygen and sulphur, which may be saturated, partially saturated or unsaturated. Examples of 4- to 14- membered heterocyclic groups include but are not limited to furan, azetidine, pyrrole, pyrrolidine, pyrazole, imidazole, triazole, isotriazole, tetrazole, thiadiazole, isothiazole, oxadiazole, pyridine, piperidine, pyrazine, oxazole, isoxazole, pyrazine, pyridazine, pyrimidine, piperazine, pyrrolidine, pyrrolidinone, morpholine, triazine, oxazine, tetrahyrofuran, tetrahydrothiophene, tetrahydrothiopyran, tetrahydropyran, 1,4-dioxane, 1,4-oxathiane, indazole, quinoline, indole, thiazole, isoquinoline, benzothiophene, benzoxazole, benzisoxazole, benzothiazole, benzisothiazole, benzofuran, dihydrobenzofuran, benzodioxole, benzimidazole or tetrahydronaphthyridine. The 4- to 14-membered heterocyclic group can be unsubstituted or substituted.
"C₃-C₁₀-cycloalkyl" denotes a fully saturated carbocyclic ring having 3 to 10 ring carbon atoms, for example a monocyclic group such as a cyclopropyl, cyclobutyl, cyclopentyl or cyclohexyl, cycloheptyl, cyclooctyl, cyclononyl or cyclodecyl, or a bicyclic group such as bicycloheptyl or bicyclooctyl.
"C₁-C₈-haloalkyl" as used herein denotes C₁-C₈-alkyl as hereinbefore defined substituted by one or more halogen atoms, preferably one, two or three halogen atoms.
"C₁-C₈-alkylamino" and "di(C₁-C₈-alkyl)amino" as used herein denote amino substituted respectively by one or two C₁-C₈-alkyl groups as hereinbefore defined, which may be the same or different.
"C₁-C₈-alkylthio" as used herein denotes straight chain or branched alkylthio having 1 to 8 carbon atoms.
"C₁-C₈-alkoxy" as used herein denotes straight chain or branched alkoxy that contains 1 to 8 carbon atoms.

Throughout this specification and in the claims that follow, unless the context requires otherwise, the word "comprise", or variations such as "comprises" or "comprising", should be understood to imply the inclusion of a stated integer or step or group of integers or steps but not the exclusion of any other integer or step or group of integers or steps.

Preferred compounds of formula I include compounds in free or salt or solvate form wherein
T¹ is a 4- to 14-membered heterocyclic group optionally substituted at one, two or three positions by R¹;
T² is a 4- to 14-membered heterocyclic group optionally substituted at one, two or three positions by R²;
K is -CR³;
either R^{a} and R^{b} are independently hydrogen;
   C₁-C₈-alkyl optionally substituted at one, two or three positions by R⁴,
   C₃-C₁₀-cycloalkyl optionally substituted at one, two or three positions by hydroxy, amino, -R¹ or R², where C₃-C₁₀-cycloalkyl is optionally fused to a phenyl group; -C(=O)-C₁-C₈-alkyl-R⁵ ;
   a 4- to 14-membered heterocyclic group optionally substituted at one, two or three positions by hydroxy, -R¹, -C(=O)-O-R¹ or -R²; or
   C₆-C₁₅-aryl optionally substituted at one, two or three positions by halo, hydroxy, - R⁵, -SO₂-R⁵ or -C(=O)-O-R¹;
or R^{a} and R^{b} together form a 4- to 14-membered heterocyclic group optionally substituted at one, two or three positions by oxo;
R¹ is C₁-C₈-alkyl optionally substituted at one, two or three positions by hydroxy;
R² is C₆-C₁₅-aryl optionally substituted at one, two or three positions by halo, hydroxy, -R¹, C₁-C₈-alkoxy, -O-C₆-C₁₅-aryl, halo-C₁-C₈-alkyl, -SO₂-NH₂ or -SO₂-C₁-C₈-alkyl;
R³ is hydrogen;
R⁴ is hydroxy, amino, -N(H)-C(=NH)-NH₂, -N(H)-SO₂-R², di(C₁-C₈-alkyl)amino, -R², -C(=O)-R⁵, -O-R², -O-R⁵, -N(H)-R⁵, -C(=O)-NH₂, -SO₂-R⁵, -C(=O)-O-R¹, -SO₂-R² or -C(=O)-N(H)-C₁-C₈-alkyl-C(=O)-N(H)-R²;
or R⁴ is a 4- to 14-membered heterocyclic group, that group being optionally substituted at one, two or three positions by hydroxy, halo, oxo, cyano, -R¹, C₁-C₈-alkoxy, C₃-C₁₀-cycloalkyl, -C(=O)-R¹, halo-C₁-C₈-alkyl, -R², -C₁-C₈-alkyl-R² or -R⁵;
or R⁴ is C₃-C₁₀-cycloalkyl substituted at one, two or three positions by hydroxy, amino, -R¹, halo-C₁-C₈-alkyl, -SO₂-NH₂ or -SO₂-R¹; and
R⁵ is a 4- to 14-membered heterocyclic group optionally substituted at one, two or three positions by oxo, amino, halo, -R¹, C₁-C₈-alkoxy, halo-C₁-C₈-alkyl, -SO₂-C₆-C₁₅-aryl or by a 4- to 14-membered heterocyclic group optionally substituted at one, two or three positions by -R¹.

Especially preferred compounds of formula I include compounds in free or salt or solvate form wherein
T¹ is a 4- to 14-membered heterocyclic group optionally substituted at one, two or three positions by R¹;
T² is a 4- to 14-membered heterocyclic group optionally substituted at one, two or three positions by R²;
K is -CR³ ;
either R^{a} and R^{b} are independently hydrogen;
   C₁-C₅-alkyl optionally substituted at one, two or three positions by R⁴,
   C₃-C₆-cycloalkyl optionally substituted at one, two or three positions by hydroxy, amino, -R¹ or R², where C₃-C₆-cycloalkyl is optionally fused to a phenyl group; -C (=O)-C₁-C₄-alkyl-R⁵ ;
   a 4- to 10-membered heterocyclic group optionally substituted at one, two or three positions by hydroxy, -R¹, -C(=O)-O-R¹ or -R²; or
   C₆-C₁₀-aryl optionally substituted at one, two or three positions by halo, hydroxy,-R⁵, -SO₂-R⁵ or -C(=O)-O-R¹;
or R^{a} and R^{b} together form a 4- to 10-membered heterocyclic group optionally substituted at one, two or three positions by oxo;
R¹ is C₁-C₄-alkyl optionally substituted at one, two or three positions by hydroxy;
R² is C₆-C₁₀-aryl optionally substituted at one, two or three positions by halo, hydroxy, -R¹, C₁-C₄-alkoxy, -O-C₆-C₁₀-aryl, halo-C₁-C₄-alkyl, -SO₂-NH₂ or -SO₂-C₁-C₄-alkyl;
R³ is hydrogen;
R⁴ is hydroxy, amino, -N(H)-C(=NH)-NH₂, -N(H)-SO₂-R², di(C₁-C₄-alkyl)amino, -R², -C(=O)-R⁵, -O-R², -O-R⁵, -N(H)-R⁵, -C(=O)-NH₂, -SO₂-R⁵, -C(=O)-O-R¹, -SO₂-R² or -C(=O)-N(H)-C₁-C₄-alkyl-C(=O)-N(H)-R²;
or R⁴ is a 4- to 10-membered heterocyclic group, that group being optionally substituted at one, two or three positions by hydroxy, halo, oxo, cyano, -R¹, C₁-C₄-alkoxy, C₃-C₆-cycloalkyl, -C(=O)-R¹, halo-C₁-C₄-alkyl, -R², -C₁-C₄-alkyl-R², or -R⁵;
or R⁴ is C₃-C₆-cycloalkyl substituted at one, two or three positions by hydroxy, amino, -R¹, halo-C₁-C₄-alkyl, -SO₂-NH₂ or -SO₂-R¹; and
R⁵ is a 4- to 10-membered heterocyclic group optionally substituted at one, two or three positions by oxo, amino, halo, -R¹, C₁-C₄-alkoxy, halo-C₁-C₄-alkyl, -SO₂-C₆-C₁₀-aryl or by a 4- to 10-membered heterocyclic group optionally substituted at one, two or three positions by -R¹.

According to formula I, the following suitable, preferred, more preferred or most preferred aspects of the invention may be incorporated independently, collectively or in any combination.

T¹ is suitably a 5- or 6-membered heterocyclic group optionally substituted at one position by C₁-C₈-alkyl or C₁-C₈-alkoxy, for example T¹ is unsubstituted pyridinyl, especially pyridin-2-yl, or pyridyl substituted by C₁-C₄-alkyl, for example 6-methyl-pyridin-2-yl.

T² is suitably a 5- or 6-membered heterocyclic group optionally substituted at one position by C₆-C₁₅-aryl optionally substituted by halo or C₁-C₄-alkyl or C₁-C₄-alkylamino, di(C₁-C₄-alkyl)-amino, For example T² is unsubstituted pyridinyl, especially unsubstituted pyridin-3-yl, or T² is pyridinyl substituted by bromo, methyl or fluoro-phenyl.

K is suitably -CR³.

R^{a} is a hydrogen.

R^{b} is suitably is C₁-C₅-alkyl optionally substituted at one position by R⁴.

R¹ is suitably C₁-C₄-alkyl optionally substituted at one position by hydroxy or halo.

R² is suitably phenyl optionally substituted at one or two positions by halo, hydroxy, -R¹, C₁-C₄-alkoxy, -O-C₆-C₁₀-aryl, halo-C₁-C₄-alkyl, -C₁-C₈-alkyl-NR⁶R⁷, -SO₂-NH2 or -SO₂-C₁-C₄-alkyl.

R³ is suitably hydrogen;

R⁴ is suitably -R² or a 4- to 10-membered heterocyclic group optionally substituted at one or two positions by hydroxy, halo, oxo, cyano, -R¹, C₁-C₄-alkoxy, C₃-C₆-cycloalkyl, -C(=O)-R¹, halo-C₁-C₄-alkyl, -R², -C₁-C₄-alkyl-R² or -R⁵.

R⁵ is suitably a 4- to 10-membered heterocyclic group optionally substituted at one or two positions by oxo, amino, halo, -R¹, C₁-C₄-alkoxy, halo-C₁-C₄-alkyl, -SO₂-phenyl or by a 5 or 6-membered heterocyclic group optionally substituted at one position by -R¹.

Compounds of formula I that contain a basic centre are capable of forming acid addition salts, particularly pharmaceutically acceptable acid addition salts. Pharmaceutically acceptable acid addition salts of the compound of formula I include those of inorganic acids, for example, hydrohalic acids such as hydrofluoric acid, hydrochloric acid, hydrobromic acid, hydroiodic acid, nitric acid, sulfuric acid, phosphoric acid; and organic acids, for example aliphatic monocarboxylic acids such as formic acid, acetic acid, trifluoroacetic acid, propionic acid and butyric acid, caprylic acid, dichloroacetic acid, hippuric acid, aliphatic hydroxy acids such as lactic acid, citric acid, tartaric acid or malic acid, gluconic acid, mandelic acid, dicarboxylic acids such as maleic acid or succinic acid, adipic acid, aspartic acid, fumaric acid, glutamic acid, malonic acid, sebacic acid, aromatic carboxylic acids such as benzoic acid, p-chloro-benzoic acid, nicotinic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid, ethanesulfonic acid, ethane-1,2-disulfonic acid, 2-hydroxy-ethanesulfonic acid, (+) camphor-10-sulfonic acid, naphthalene-2-sulfonic acid, naphthalene-1,5-disulfonic acid or p-toluenesulfonic acid. These salts may be prepared from compounds of formula I by known salt-forming procedures. Pharmaceutically acceptable solvates are generally hydrates.

Compounds of formula I which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine, arginine, benethamine, benzathine, diethanolamine, 4-(2-hydroxyethyl)morpholine,1-(2-hydroxyethyl)pyrrolidine, N -methyl glucamine, piperazine, triethanolamine or tromethamine. These salts may be prepared from compounds of formula I by known salt-forming procedures. Compounds of formula I that contain acidic, e.g. carboxyl, groups may also exist as zwitterions with the quaternary ammonium centre.

Compounds of formula I in free form may be converted into salt form, and vice versa, in a conventional manner. The compounds in free or salt form can be obtained in the form of hydrates or solvates containing a solvent used for crystallisation. Compounds of formula I can be recovered from reaction mixtures and purified in a conventional manner. Isomers, such as enantiomers, may be obtained in a conventional manner, e.g. by fractional crystallisation or asymmetric synthesis from correspondingly asymmetrically substituted, e.g. optically active, starting materials.

Many compounds of the invention contain at least one asymmetric carbon atom and thus they exist in individual optically active isomeric forms or as mixtures thereof, e.g. as racemic mixtures. In cases where additional asymmetric centres exist the present invention also embraces both individual optically active isomers as well as mixtures, e.g. diastereomeric mixtures, thereof.

The invention includes all such forms, in particular the pure isomeric forms. The different isomeric forms may be separated or resolved one from the other by conventional methods, or any given isomer may be obtained by conventional synthetic methods or; by stereospecific or asymmetric syntheses. Since the compounds of the invention are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1 %, more suitably at least 5% and preferably from 10 to 59% of a compound of the invention.

Specific especially preferred compounds of the invention are those described hereinafter in the Examples.

The invention also provides a process for the preparation of compounds of formula I which comprises reacting a compound of formula II 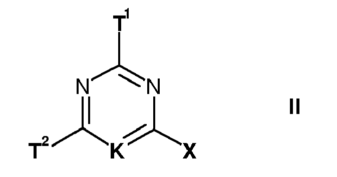 where T¹, T² and K are as hereinbefore defined and X is halo, with a compound of formula III 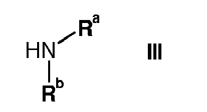 where R^{a} and R^{b} are as hereinbefore defined. This may be effected using known procedures for reacting halogenated heterocyclic groups with primary or secondary amines or analogously as hereinafter described in the Examples. The reaction is conveniently carried out in an organic solvent, for example N -methylpyrrolidinone (NMP), optionally in the presence of an inorganic base, for example potassium carbonate. Suitable reaction temperatures are elevated temperatures, e.g. from 100° C to 150° C, preferably by microwaving at about 120° C.

Compounds of formula II are formed by reacting a compound of formula IV 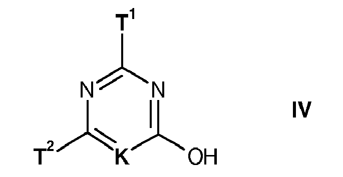 where T¹, T² and K are as hereinbefore defined with a halogenating agent. This may be effected using known procedures for halogenating hydroxy compounds or analogously as hereinafter described in the Examples. The halogenating agent is preferably a combination of a strong Lewis acid e.g. POCl₃ and PCl₅. Suitable reaction temperatures are elevated temperatures, for example reflux temperature.

Compounds of formula III are known or may be prepared by known procedures.

Compounds of formula IV are formed by reacting a compound of formula V 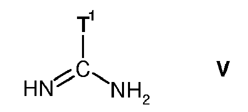 where T¹ is as hereinbefore defined with a compound of formula VI 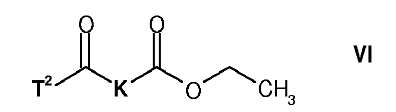 where T² and K are as hereinbefore defined. This may be effected using known procedures for reacting amidine with a β-ketoester or analogously as hereinafter described in the Examples. The reaction is conveniently carried out in an organic solvent, for example ethanol, preferably in the presence of an inorganic base sodium hydroxide. Suitable reaction temperatures are from 0° C to 50° C, conveniently room temperature.

Compounds of formula V or VI are known or may be prepared by known procedures.

Compounds of formula I in pharmaceutically acceptable salt, hereinafter referred to alternatively as "agents of the invention", are useful as pharmaceuticals. Accordingly the invention also provides a compound of formula I in pharmaceutically acceptable salt for use as a pharmaceutical. The agents of the invention act as activin-like kinase ("ALK")-5 inhibitors . At least many agents of the invention also act as ALK-4 inhibitors too.

TGF-β1 is the prototypic member of a family of cytokines including the TGF-βs, activins, inhibins, bone morphogenetic proteins and Mullerian-inhibiting substance, that signal through a family of single transmembrane serine/threonine kinase receptors. These receptors can be divided into two classes, the type I or activin like kinase (ALK) receptors and type II receptors. The ALK receptors are distinguished from the type II receptors in that the ALK receptors (a) lack the serine/threonine rich intracellular tail, (b) possess serine/threonine kinase domains that are very homologous between type I receptors, and (c) share a common sequence motif called the GS domain, consisting of a region rich in glycine and serine residues. The GS domain is at the amino terminal end of the intracellular kinase domain and is critical for activation by the type II receptor. Several studies have shown that TGF-β signalling requires both the ALK and type II receptors. Specifically, the type II receptor phosphorylates the GS domain of the type I receptor for TGF-β, ALK5, in the presence of TGF-β. The ALK5, in turn, phosphorylates the cytoplasmic proteins smad2 and smad3 at two carboxy terminal serines. The phosphorylated smad proteins translocate into the nucleus and activate genes that contribute to the production of extracellular matrix. Therefore, preferred compounds of this invention are selective in that they inhibit the type I receptor.

Activins transduce signals in a manner similar to TGF-β. Activins bind to serine/thereonine kinase, the activin type II receptor (ActRIIB), and the activated type II receptor hyper-phosphorylates serine/threonine residues in the GS region of the ALK4. The activated ALK4 in turn phosphorylates Smad2 and Smad3. The consequent formation of a hetero-Smad complex with Smad4 results in the activin-induced regulation of gene transcription.

Activation of the TGF-β1 axis and expansion of extracellular matrix are early and persistent contributors to the development and progression of chronic renal disease and vascular disease. Border W.A., et al, N. Engl. J. Med., 1994; 331(19), 1286-92. Further, TGF-β1 plays a role in the formation of fibronectin and plasminogen activator inhibitor-1, components of sclerotic deposits, through the action of smad3 phosphorylation by the TGF-β1 receptor ALK5. Zhang Y., et al, Nature, 1998; 394(6696), 909-13; Usui T., et al, Invest. Ophthalmol. Vis. Sci., 1998; 39(11), 1981-9.

Progressive fibrosis in the kidney and cardiovascular system is a major cause of suffering and death and an important contributor to the cost of health care. TGF-β1 has been implicated in many renal fibrotic disorders. Border W.A., et al, N. Engl. J. Med., 1994; 331(19),1286-92. TGF-β1 is elevated in acute and chronic glomerulonephritis Yoshioka K., et al, Lab. Invest., 1993; 68(2),154-63, diabetic nephropathy Yamamoto, T., et al, 1993, PNAS 90, 1814-1818., allograft rejection, HIV nephropathy and angiotensin-induced nephropathy Border W.A., et al, N. Engl. 5 J. Med., 1994; 331(19), 1286-92. In these diseases the levels of TGF-β1 expression coincide with the production of extracellular matrix. Three lines of evidence suggest a causal relationship between TGF-β1 and the production of matrix. First, normal glomeruli, mesangial cells and non-renal cells can be induced to produce extracellular-matrix protein and inhibit protease activity by exogenous TGF-β1 in vitro. Second, neutralizing antibodies against TGF-β1 can prevent the accumulation of extracellular matrix in nephritic rats. Third, TGF-β1 transgenic mice or *in vivo* transfection of the TGF-β1 gene into normal rat kidneys resulted in the rapid development of glomerulosclerosis. Kopp J.B., et al, Lab. Invest., 1996; 74(6),991 1003. Thus, inhibition of TGF-β1 activity is indicated as a therapeutic intervention in chronic renal disease.

TGF-β1 and its receptors are increased in injured blood vessels and are indicated in neointima formation following balloon angioplasty Saltis J., et al, Clin. Exp. Pharmacol. Physiol., 1996; 23(3),193-200. In addition TGF-β1 is a potent stimulator of smooth muscle cell (" SMC") migration in vitro and migration of SMC in the arterial wall is a contributing factor in the pathogenesis of atherosclerosis and restenosis. Moreover, in multivariate analysis of the endothelial cell products against total cholesterol, TGF-β receptor ALK5 correlated with total cholesterol (P < 0.001) Blann A.D., et al, A therosclerosis, 1996; 120(1-2), 221-6. Furthermore, SMC derived from human atherosclerotic lesions have an increased ALK5/ TGF-β type 11 receptor ratio. Because TGF-β1 is over-expressed in fibroproliferative vascular lesions, receptor- I variant cells would be allowed to grow in a slow, but uncontrolled fashion, while overproducing extracellular matrix components McCaffrey T.A., et al, Jr., J. Clin.; Invest., 1995; 96(6), 2667-75. TGF-β1 was immunolocalized to non-foamy macrophages in atherosclerotic lesions where active matrix synthesis occurs, suggesting that non-foamy macrophages may participate in modulating matrix gene expression in atherosclerotic remodelling via a TGF-β-dependent mechanism. Therefore, inhibiting the action of TGF-β1 on ALK5 is also indicated in atherosclerosis and restenosis.

TGF-β1 is also indicated in wound repair. Neutralizing antibodies to TGF-β1 have been used in a number of models to illustrate that inhibition of TGF-β1 signalling is beneficial in restoring function after injury by limiting excessive scar formation during the healing process. For example, neutralizing antibodies to TGF-β1 and TGF-β2 reduced scar formation and improved the cytoarchitecture of the neodermis by reducing the number of monocytes and macrophages as well as decreasing dermal fibronectin and collagen deposition in rats Shah M., J. Cell. Sci., 1995,108, 985-1002. Moreover, TGF-β antibodies also improve healing of corneal wounds in rabbits Moller-Pedersen T., Curr. Eye Res., 1998,17, 736-747, and accelerate wound healing of gastric ulcers in the rat, Ernst H., Gut, 1996, 39, 172-175. These data strongly suggest that limiting the activity of TGF-β would be beneficial in many tissues and suggest that any disease with chronic elevation of TGF-β would benefit by inhibiting smad2 and smad3 signalling pathways.

TGF-β is also implicated in peritoneal adhesions Sand G.M., et al, Wound Repair Regeneration, 1999 Nov-Dec, 7(6), 504-510. Therefore, inhibitors of ALK5 would be beneficial in preventing peritoneal and sub-dermal fibrotic adhesions following surgical procedures.

TGF-β is also implicated in photoaging of the skin (see Fisher GJ. Kang SW. Varani J. Bata-Csorgo Z. Wan YS. Data S. Voorhees J J., Mechanisms of photoaging and chronological skin ageing, Archives of Dermatology, 138(11):1462- 1470, 2002 Nov. and Schwartz E. Sapadin AN. Kligman LH. "Ultraviolet B radiation increases steady state mRNA levels for cytokines and integrins in hairless mouse skin- modulation by 25 topical tretinoin", Archives of Dermatological Research, 290(3):137-144, 1998 Mar.)
TGF-β signalling is also implicated in the development of pulmonary disorders, in particular pulmonary hypertension and pulmonary fibrosis (see Morrell NW, Yang X, Upton PD, Jourdan KB, Morgan N, Sheares KK, Trembath RC., Altered growth responses of pulmonary artery smooth muscle cells from patients with primary pulmonary hypertension to transforming growth factor-beta(1) and bone morphogenetic proteins. Circulation. 2001 Aug 14;104(7):790-5. Bhatt N, Baran CP, Allen J, Magro C, Marsh CB., Promising pharmacologic innovations in treating pulmonary fibrosis. Curr O pin Pharmacol. 2006 Apr 28).

TGF-β1 levels are increased in animal models of pulmonary hypertension (Mata-Greenwood E, Meyrick B, Steinhorn RH, Fineman JR, Black SM. Alterations in TGF-betal expression in lambs with increased pulmonary blood flow and pulmonary hypertension. Am. J. Physiol. Lung Cell Mol. Physiol. 2003 Jul; 285(1):L209-21). Other studies have suggested that pulmonary endothelial cell-derived TGF-β1 can stimulate the growth of pulmonary vascular smooth muscle cells which may underlie the enhanced muscularisation observed in the pulmonary vasculature of individuals with pulmonary hypertension (Sakao S, Taraseviciene-Stewart L, Wood K, Cool CD, Norbert VF. Apoptosis of pulmonary microvascular endothelial cells stimulates vascular smooth muscle cell growth. Am. J. Physiol. Lung Cell Mol. Physiol. 2006 Apr 14). Therefore, inhibiting the action of TGF-β1 on ALK5 is indicated as a therapeutic intervention in pulmonary hypertension.

Additionally, dys-regulated TGF-β signalling has also been implicated in the development of idiopathic pulmonary fibrosis. Activation of ALK5 results in Smad3-activation and downstream modulation of the expression of genes involved in the fibrotic process such as plasminogen activator inhibitor-1, pro-collagen 3A1, and connective tissue growth factor. The levels of TGF-β1 and its downstream pro-fibrotic mediators have been demonstrated to be up-regulated in bronchoalveolar lavage taken from patients with idiopathic pulmonary fibrosis (Hiwatari N, Shimura S, Yamauchi K, Nara M, Hida W, Shirato K. Significance of elevated procollagen-III-peptide and transforming growth factor-beta levels of bronchoalveolar lavage fluids from idiopathic pulmonary fibrosis patients. Tohoku J. Exp. Med. 1997 Feb; 181(2): 285-95) and in animal models of idiopathic pulmonary fibrosis (Westergren-Thorsson G, Hernnas J, Sarnstrand B, Oldberg A, Heinegard D, Malmstrom A. Altered expression of small proteoglycans, collagen, and transforming growth factor-beta 1 in developing bleomycin-induced pulmonary fibrosis in rats. J. Clin. Invest. 1993 Aug;92(2):632-7).

Transient over-expression of active TGF-β1 in murine lungs, using adenoviral vector-mediated gene transfer, resulted in progressive pulmonary fibrosis in wild-type mice, whereas no fibrosis was seen in the lungs of Smad3 knockout mice up to 28 days following TGF-β1 challenge (Khalil N, Parekh TV, O'Connor RN, Gold LI. Differential expression of transforming growth factor-beta type I and II receptors by pulmonary cells in bleomycin-induced lung injury: correlation with repair and fibrosis. Exp. Lung. Res. 2002 Apr-May;28(3):233-50. Thus, inhibition of TGF-β1 activation of ALK5 is also indicated for pulmonary fibrosis.

Activin signaling and overexpression of activin is linked to pathological disorders that involve extracellular matrix accumulation and fibrosis (e.g., Matsuse, T. et al., Am. J. Respir Cell Mol. Biol. 13:17-24 (1995); Inoue, S. et al., Biochem. Biophys. Res. Comn. 205:441- 448 (1994); Matsuse, T. et al., Am. J. Pathol. 148:707-713 (1996); De Bleser et al., Hepatology 26:905-912 (1997); Pawlowski, J. E., et al., J. Clin. Invest. 100:639-648 (1997); Sugiyama, M. et al., Gastroenterology 114:550-558 (1998); Munz, B. et al., EMBO J. 18:5205-5215 (1999)), inflammatory responses (e.g., Rosendahl, A. et al., Am. J. Respir. Cell Mol. Biol. 25:60-68 (2001), cachexia or wasting (Matzuk7 M. M. et al., Proc. Natl. Acad. Sci. USA 91:8817-8821 (1994); Coerver, K. A. et al., Mol. Endocrinol. 10:531 543 (1996); Cipriano, S. C. et al., Endocrinology 141:2319-2327 (2000)), diseases or pathological responses in the central nervous system (e.g., Logan, A. et al., Eur. J Neurosci. 11:2367- 2374 (1999); Logan, A. et al., Exp. Neurol. 159:504-510 (1999); Masliah, E. et al., Neurochem. Int. 39:393-400 (2001); De Groot, C. J. A. et al., J Neuropathol. Exp. Neural. 58:174-187 (1999); John, G. R. et al., Nat. Med. 8:1115-1121 (2002)) and hypertension (e.g., Dahly, A. J. et al., Am. J. Physiol. Regul. Integr Comp. Physiol. 283: R757-767 (2002)). Studies have shown that TGF-β and activin can act synergistically to induce extracellular matrix production (e.g., Sugiyama, M. et al., Gastroerterology 114; 550-558 (1998)).

It follows, therefore, that inhibition of ALK5 and/or ALK4 phosphorylation of Smad2 and Smad3 by the compounds of the present invention can be useful to treat and prevent disorders that involve these signaling pathways.

Activin signalling is also implicated in the development of pulmonary disorders, in particular pulmonary hypertension and pulmonary fibrosis. For example, the expression of activin A in lung samples from patients with interstitial pulmonary fibrosis demonstrated strong expression of activin A on metaplastic epithelium, hyperplastic smooth muscle cells, desquamated cells, and alveolar macrophages. Pulmonary arteries from patients with primary or secondary pulmonary hypertension showed abundant immunoreactive activin A on smooth muscle cells. These findings suggest a potential role for this growth factor, activin A, in the pathogenesis of pulmonary tissue remodeling associated with interstitial pulmonary fibrosis and pulmonary hypertension (Matsuse T, Ikegami A, Ohga E, Hosoi T, Oka T, Kida K, Fukayama M, Inoue S, Nagase T, Ouchi Y, Fukuchi Y. Expression of immunoreactive activin A protein in remodeling lesions associated with interstitial pulmonary fibrosis. Am. J. Pathol. 1996 Mar;148(3):707-13). An increase in fibroblasts and associated connective tissue is a feature of pulmonary fibrosis and pulmonary hypertension. Activin A has been demonstrated to modulate human lung fibroblast (HFL1) activity, particularly with respect to proliferation and its differentiation into myofibroblast, thus activin A has potential effects on proliferation of lung fibroblast and its differentiation into myofibroblast, and may contribute to structural remodeling observed in pulmonary fibrosis and hypertension (Ohga E, Matsuse T, Teramoto S, Katayama H, Nagase T, Fukuchi Y, Ouchi Y. Effects of activin A on proliferation and differentiation of human lung fibroblasts. Biochem. Biophys. Res. Commun. 1996 Nov 12;228(2):391-6). The induction of pulmonary fibrosis mediated by bleomycin challenge in rats results in the up-regulated expression of activin A in macrophages infiltrated in the lung, and was detected in fibroblasts accumulated in the fibrotic area. Administration of follistatin, an antagonist of activin signalling to bleomycin-treated rats significantly reduced the number of macrophages and neutrophils in bronchoalveolar lavage and reduced the protein content. Follistatin markedly reduced the number of infiltrating cells, ameliorated the destruction of lung architecture, and attenuated lung fibrosis (Aoki F, Kurabayashi M, Hasegawa Y, Kojima I. Attenuation of bleomycin-induced pulmonary fibrosis by follistatin. Am. J Respir. Crit. Care Med. 2005 Sep 15;172(6):713-20).

Therefore, inhibiting activin signalling via ALK4 inhibition may also be beneficial for the treatment of pulmonary fibrosis and pulmonary hypertension.

It has been demonstrated recently that reduction in TGF-β signaling, through its effector Smad3, enhances the mechanical properties and mineral concentration of the bone matrix, as well as the bone mass, enabling the bone to better resist fracture. These results suggest that reduction of TGF-β signaling could be considered as a therapeutic target to treat bone disorders. (Balooch G, et al. Proc. Natl. Acad. Sci. U S A. 2005 Dec 27;102(52):18813-8). Thus, inhibition of TGF-β1 activation of ALK5 is also indicated for increasing mineral density strength and content of bone and may be utilized to treat a wide variety of conditions, including for example, osteopenia, osteoporosis, fractures and other disorders in which low bone mineral density are a hallmark of the disease.

Having regard to their inhibition of ALK-5 and/or ALK-4 receptors, agents of the invention are useful in the treatment of conditions mediated by the ALK-5 and/or ALK-4 receptors. Treatment in accordance with the invention may be symptomatic or prophylactic.

Therefore according to a further aspect, the invention provides the use of a compound defined in the first aspect in the preparation of a medicament for treating or preventing a disease or condition mediated by ALK-5 inhibition or ALK-4 inhibition.

Diseases or condition mediated by ALK-5 inhibition or ALK-4 inhibition include glomerulonephritis, diabetic nephropathy, lupus nephritis, hypertension-induced nephropathy, renal interstitial fibrosis, renal fibrosis resulting from complications of drug exposure, HIV-associated nephropathy, transplant necropathy, liver fibrosis due to all etiologies, hepatic dysfunction attributable to infections, alcohol- induced hepatitis, disorders of the biliary tree, pulmonary fibrosis, pulmonary hypertension, acute lung injury, adult respiratory distress syndrome, idiopathic pulmonary fibrosis, chronic obstructive pulmonary disease, pulmonary disease due to infectious or toxic agents, post-infarction cardiac fibrosis, congestive heart failure, dilated cardiomyopathy, myocarditis, vascular stenosis, restenosis, atherosclerosis, ocular scarring, corneal scarring, proliferative vitreoretinopathy, excessive or hypertrophic scar or keloid formation in the dermis occurring during wound healing resulting from trauma or surgical wounds, peritoneal and sub dermal adhesion, scleroderma, fibrosclerosis, progressive systemic sclerosis, dermatomyositis, polymyositis, arthritis, ulcers, impaired neurological function, male erectile dysfunction, Alzheimer's disease, Raynaud's syndrome, fibrotic cancers, tumor metastasis growth, radiation-induced fibrosis, thrombosis, and bone conditions such as osteopenia and osteoporosis, which are associated with increased calcium depletion or resorption or in which stimulation of bone formation and calcium fixation in the bone is desirable.

Diseases or conditions mediated by ALK-5 inhibition in particular include chronic renal disease, acute renal disease, wound healing, arthritis, osteoporosis, kidney disease, congestive heart failure, inflammatory or obstructive airways diseases, pulmonary hypertension, ulcers (including diabetic ulcers, chronic ulcers, gastric ulcers, and duodenal ulcers), ocular disorders, corneal wounds, diabetic nephropathy, impaired neuro-logical function, Alzheimer's disease, atherosclerosis, peritoneal and sub-dermal adhesion, any disease wherein fibrosis is a major component, including, but not limited to kidney fibrosis, lung fibrosis and liver fibrosis, for example, hepatitis B virus (HBV), hepatitis C virus (HCV), alcohol-induced hepatitis, haemochromatosis, primary biliary cirrhosis, restenosis, retroperitoneal fibrosis, mesenteric fibrosis, endometriosis, keloids, cancer, abnormal bone function, inflammatory disorders, scarring and photaging of the skin.

Inflammatory or obstructive airways diseases to which the present invention is applicable include asthma of whatever type or genesis including both intrinsic (non-allergic) asthma and extrinsic (allergic) asthma. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and
characterised by asthma attack, e.g. between the hours of about 4 to 6 am, i.e. at a time normally substantially distant from any previously administered symptomatic asthma therapy.

Other inflammatory or obstructive airways diseases and conditions to which the present invention is applicable include adult/acute respiratory distress syndrome (ARDS), chronic obstructive pulmonary or airways disease (COPD or COAD), including chronic bronchitis, or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Preferably the disease or condition mediated by ALK-5 inhibition or ALK-4 inhibition is pulmonary hypertension, pulmonary fibrosis or osteoporosis.

Pulmonary hypertension to be treated in accordance with the invention includes primary pulmonary hypertension (PPH); secondary pulmonary hypertension (SPH); familial PPH; sporadic PPH; precapillary pulmonary hypertension; pulmonary arterial hypertension (PAH); pulmonary artery hypertension; idiopathic pulmonary hypertension; thrombotic pulmonary arteriopathy (TPA); plexogenic pulmonary arteriopathy; functional classes I to IV pulmonary hypertension; and pulmonary hypertension associated with, related to, or secondary to, left ventricular dysfunction, mitral valvular disease, constrictive pericarditis, aortic stenosis, cardiomyopathy, mediastinal fibrosis, anomalous pulmonary venous drainage, pulmonary venoocclusive disease, collagen vasular disease, congenital heart disease, HIV virus infection, drugs and toxins such as fenfluramines, congenital heart disease, pulmonary venous hypertension, chronic obstructive pulmonary disease, interstitial lung disease, sleep-disordered breathing, alveolar hypoventilation disorder, chronic exposure to high altitude, neonatal lung disease, alveolar-capillary dysplasia, sickle cell disease, other coagulation disorder, chronic thromboemboli, connective tissue disease, lupus, schistosomiasis, sarcoidosis or pulmonary capillary hemangiomatosis.

Pulmonary hypertension to be treated in accordance with the invention is most particularly pulmonary hypertension associated with disorders of the respiratory system and/or hypoxemia, including chronic obstructive pulmonary disease, interstitial lung disease, sleep-disordered breathing, alveolar hypoventilation disorders, chronic exposure to high altitude, neonatal lung disease and alveolar-capillary dysplasia, but especially chronic obstructive pulmonary disease.

Lung fibrosis includes idiopathic pulmonary fibrosis in particular.

Osteoporosis is a systemic skeletal disorder characterized by low bone mass and micro-architectural deterioration of bone tissue, with a consequent increase in bone fragility and susceptibility to fracture. The osteoporotic syndrome is multi faceted, encompassing primary disorders such as postmenopausal or age-related osteporosis, and secondary conditions that accompany disease states or medications. The mechanical properties and composition of bone matrix, along with bone mass and architecture, are critical determinants of a bone's ability to resist fracture.

Thus in a further aspect the invention includes a method for preventing or treating bone conditions which are associated with increased calcium depletion or resorption or in which stimulation of bone formation and calcium fixation in the bone is desirable in which an effective amount of a compound of formula I as defined above, or a pharmaceutically-acceptable and -cleavable ester, or acid addition salt thereof is administered to a patient in need of such treatment.

In a yet further aspect the invention includes a pharmaceutical composition for preventing or treating bone conditions which are associated with increased calcium depletion or resorption or in which stimulation of bone formation and calcium fixation in the bone is desirable comprising a compound of formula I as defined above, or a pharmaceutically-acceptable and -cleavable ester, or acid addition salt thereof, in admixture with a pharmaceutically acceptable excipient, diluent or carrier.

The compounds of the Examples herein below generally have IC₅₀ values below 2 µM, and mostly below 1 µM. For instance, the compounds of Examples 1, 2, 3 and 4 have IC₅₀ values of 0.43, 0.79, 0.99 and 1.12 respectively.

The kinase activity of ALK5 is assessed by measuring radiolabelled phosphate [33P] incorporation in to the generic substrate, casein. The kinase domain of human ALK5 (amino acids 200-503) is fused to an N-terminal histidine tag. The kinase activity of ALK5 is rendered constitutive via point mutation at amino acid 204 (threonine to aspartate modification, ALK5 T204D) and the kinase construct is engineered to be expressed from a baculovirus expression construct in insect cells. The purified, recombinantly-expressed histidine-tagged ALK5 T204D protein is dissolved at 5.4 mg/ml in 50 mM Tris-HCl pH 8.0, 150 mM NaCl, 5 mM DTT. ALK5 T204D is dissolved to 2.5 µg/ml in assay buffer (Assay buffer: 20 mM Tris-HCl pH 7.4, 10 mM MgCl₂, 2 mM MnCl₂) on the day of use.

Test compounds and reference compounds are dissolved in assay buffer without DTT containing 5% (v/v) DMSO. Stock solutions of test and reference compounds are diluted in assay buffer with DTT (1.25 mM) containing 4.5% (v/v) DMSO. 10 µl of test or reference compound are added to the appropriate wells of 96 well U-bottomed plate. Total enzyme activity is determined by measuring ALK5 T204D activity in the absence of ALK5 kinase inhibitor reference compounds. Non-specific binding (NSB) is determined by measuring the activity of ALK5 T204D in the presence of ALK5 kinase inhibitor reference compounds. 10 µl of dephosphorylated casein stock solution (dephosphorylated casein is dissolved in ddH₂O at 20 mg/ml) is added per well (200 µg/well final assay concentration). 20 µl of ALK5 T204D (2.5 µg/ml solution) is added per well (50 ng/well final assay concentration). The plates are left to incubate at room temperature for 10 minutes.

10 µl of ATP mix is added to the well to initiate the reaction (0.66 nM [³³P]ATP/1 µM unlabelled ATP/well final assay concentration). The ATP mix is prepared as follows, unlabelled ATP (3 mM) is dissolved in ddH₂O and pH adjusted to 7.4. The stock concentration of [³³P]ATP is 10 µCi/µl. The appropriate volume of [³³P]ATP is added to unlabelled ATP solution such that the final assay concentration per well is 0.1 µCi. Following addition of the ATP mix, the plates are incubated at room temperature for 50 minutes. The kinase reaction is terminated by the addition of 50 µL Stop Buffer (20 mM Tris-HC1 pH 7.4, 10 mM EDTA).

75 µl/well from the reaction plate is transferred to a Multiscreen-IP plate (MultiScreen-IP plates are prepared by added 50 µL of 70% (v/v) ethanol per well and incubated for 5 minutes at room temperature. The ethanol is removed by aspiration via a MultiScreen HTS Vaccum Manifold unit (Millipore, Cat no: MSVMHT500). The plates are washed twice by adding 200 µl/well ddH₂O). The MultiScreen-IP plate is incubated at room temperature for 30 minutes to allowing binding of casein to the plate. The MultiScreen-IP plates are washed three times by adding 200 µl/well 100 mM phosphoric acid solution and the gasket is carefully removed from the back of the MultiScreen-IP plate and the plate dried in the oven for 30 minutes. The MultiScreen-IP plate is backsealed, 50 µL of Microscint™20 is added, then the plates are topsealed and radiolabelled casein detected and quantified on a TopCount™ plate-reader using the ³³P scintillation protocol.

The agents of the invention are also useful as co-therapeutic agents for use in combination with other drug substances such as anti-inflammatory, bronchodilatory, antihistamine, decongestant or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with one or more other drug substances in a fixed pharmaceutical composition or it may be administered separately, before, simultaneously with or after the other drug substance(s).

Such anti-inflammatory drugs include steroids, in particular glucocorticosteroids such as budesonide, beclamethasone dipropionate, fluticasone propionate, ciclesonide or mometasone furoate and compounds described in WO 0200679, WO 0288167, WO 0212266 and WO 02100879; LTB4 antagonists such as those described in US 5451700; LTB4 antagonists such as those described in US 5451700; LTD4 antagonists such as montelukast and zafirlukast; dopamine receptor agonists such as cabergoline, bromocriptine, ropinirole and 4-hydroxy-7-[2-[[2-[[3-(2-phenylethoxy)-propyl]sulfonyl]ethyl]amino]ethyl]-2(3H)-benzothiazolone and pharmaceutically acceptable salts thereof (the hydrochloride being Viozan® - AstraZeneca); PDE4 inhibitors such as Ariflo® (GlaxoSmith Kline), Roflumilast (Byk Gulden),V-11294A (Napp), BAY19-8004 (Bayer), SCH-351591 (Schering-Plough), Arofylline (Almirall Prodesfarma), PD189659 (Parke-Davis), AWD-12-281 (Asta Medica), CDC-801 (Celgene) and KW-4490 (Kyowa Hakko Kogyo); A2a agonists such as those described in EP 1052264, EP 1241176, WO 0023457, WO0077018, WO 0123399, WO 0160835, WO 0194368, WO 0200676, WO 0222630, WO 0296462, WO 0127130, WO 0127131, WO 9602543, WO 9602553, WO 9828319, WO 9924449, WO 9924450, WO 9924451, WO 9938877, WO 9941267, WO 9967263, WO 9967264, WO 9967265, WO 9967266, WO 9417090, EP 409595A2 and WO 0078774; and A2b antagonists such as those described in WO 02/42298.

Such bronchodilatory drugs include beta-2 adrenoceptor agonists. Suitable beta-2 adrenoceptor agonists include albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound of formula 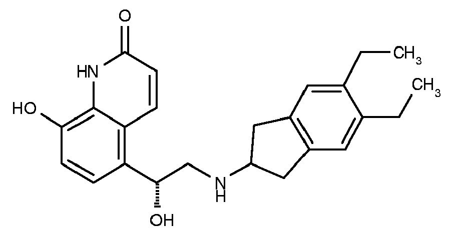 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, US 2005/0133417, US 2005/5159448, WO O1/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 , WO 04/80964, EP1460064, WO 04/087142, WO 04/089892, EP 01477167, US 2004/0242622, US 2004/0229904, WO 04/108675, WO 04/108676, WO 05/033121, WO 05/040103, WO 05/044787, WO 05/058867, WO 05/065650, WO 05/066140 and WO 05/07908.

Such bronchodilatory drugs also include other anticholinergic or antimuscarinic agents, such as those described in EP 424021, US 5171744 (Pfizer) and WO 01/04118 (Almirall Prodesfarma) and but in particular ipratropium bromide, oxitropium bromide and tiotropium bromide, and beta-2 adrenoceptor agonists such as salbutamol, terbutaline, salmeterol and, especially, formoterol and pharmaceutically acceptable salts thereof.

Suitable dual anti-inflammatory and bronchodilatory drugs include dual beta-2 adrenoceptor agonist / muscarinic antagonists such as those disclosed in US 2004/0167167, US 2004/0242622, US 2005/182092, WO 04/74246 and WO 04/74812.

Suitable antihistaminic/anti-allergic drug substances include acetaminophen, activastine, astemizole, azelastin, bamipin, cetirizine hydrochloride, cexchloropheniramine, chlorophenoxamine, clemastine fumarate, desloratidine, dimenhydrinate, dimetinden, diphenhydramine, doxylamine, ebastine, emedastin, epinastine, fexofenadine hydrochloride, ketotifen, levocabastin, loratidine, meclizine, mizolastine, pheniramine, promethazine and tefenadine, as well as those disclosed in JP 2004107299, WO 03/099807 and WO 04/026841 (including any pharmacologically acceptable acid addition salts thereof which may exist).

According to a further embodiment of the invention, the agents of the Invention may be employed as adjunct or adjuvant to other therapy, e.g. a therapy using a bone resorption inhibitor, for example as in osteoporosis therapy, in particular a therapy employing calcium, a ealeitonin or an analogue or derivative thereof, e.g. salmon, eel or human calcitonin, a steroid hormone, e.g. an estrogen, a partial estrogen agonist or estrogen-gestagen combination, a SERM (Selective Estrogen Receptor Modulator) e.g. raloxifene, lasofoxifene, TSE-424, FC1271, Tibolone (Livial A), vitamin D or an analog thereof or PTH, a PTH fragment or a PTH derivative e.g. PTH (1-84), PTH (1-34), PTH (1-36), PTH (1-38), PTH (1-31)NH2 or PTS 893.

In accordance with the foregoing, the present invention also provides a method for the treatment of an obstructive or inflammatory airways disease which comprises administering to a subject, particularly a human subject, in need thereof a compound of formula I, or a pharmaceutically acceptable salt or solvate thereof, as hereinbefore described. In another aspect, the invention provides a compound of formula I, or a pharmaceutically acceptable salt or solvate thereof, as hereinbefore described for use in the preparation of a medicament for the treatment of an obstructive or inflammatory airways disease.

The agents of the invention may be administered by any appropriate route, e.g. orally, for example in the form of a tablet or capsule; parenterally, for example intravenously; topically to the skin, for example in the treatment of psoriasis; intranasally, for example in the treatment of hay fever; or, preferably, by inhalation, particularly in the treatment of obstructive or inflammatory airways diseases. In particular, the agents of the invention may be delivered as an inhalable formulation for the treatment of COPD and asthma.

In a further aspect, the invention also provides a pharmaceutical composition comprising a compound of formula I in free form or in the form of a pharmaceutically acceptable salt or solvate thereof, optionally together with a pharmaceutically acceptable diluent or carrier therefor. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

Where the inhalable form of the active ingredient is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy. For example, an aerosol composition may be administered from a coated can, for example as described in EP-A-0642992. Where the inhalable form of the active ingredient is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer, sometimes referred to as a soft mist or soft spray inhaler, for example an electronically controlled device such as an AERx (Aradigm, US) or Aerodose (Aerogen), or a mechanical device such as a RESPIMAT (Boehringer Ingelheim) nebulizer which allows much smaller nebulized volumes, e.g. 10 to 100 µl, than conventional nebulizers. Where the inhalable form of the active ingredient is the finely divided particulate form, the inhalation device may be, for example, a dry powder inhalation device adapted to deliver dry powder from a capsule or blister containing a dry powder comprising a dosage unit of (A) and/or (B) or a multidose dry powder inhalation (MDPI) device adapted to deliver, for example, 3-25 mg of dry powder comprising a dosage unit of (A) and/or (B) per actuation. The dry powder composition preferably contains a diluent or carrier, such as lactose, and a compound that helps to protect against product performance deterioration due to moisture e.g. magnesium stearate. Suitable such dry powder inhalation devices include devices disclosed in US 3991761 (including the AEROLIZER™ device), WO 05/113042, WO 97/20589 (including the CERTIHALER™ device), WO 97/30743 (including the TWISTHALER™ device) and WO 05/37353 (including the GYROHALER™ device).

The invention also includes (A) a compound of formula I as hereinbefore described in free form, or a pharmaceutically acceptable salt or solvate thereof, in inhalable form; (B) an inhalable medicament comprising such a compound in inhalable form together with a pharmaceutically acceptable carrier in inhalable form; (C) a pharmaceutical product comprising such a compound in inhalable form in association with an inhalation device; and (D) an inhalation device containing such a compound in inhalable form.

Dosages of agents of the invention employed in practising the present invention will of course vary depending, for example, on the particular condition to be treated, the effect desired and the mode of administration. In general, suitable daily dosages for administration by inhalation are of the order of 0.0001 to 30 mg/kg, typically 0.01 to 10 mg per patient, while for oral administration suitable daily doses are of the order of 0.01 to 100 mg/kg.

The invention is illustrated by the following Examples.

Especially preferred compounds of the present invention include compounds of formula VII 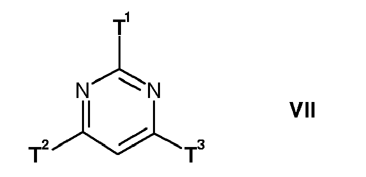 where T¹, T² and T³ are as shown in Table I below. The method of preparation being described
LCMS are recorded on an Agilent 1100 LC system with a Waters Xterra MS C18 4.6 x 100 5 µM column, eluting with 5-95% 10 mM aqueous ammonium bicarbonate in acetonitrile over 2.5 minutes, with negative ion electrospray ionization or 5-95% water + 0.1% TFA in acetonitrile with positive ion electrospray ionization. [M+H]⁺ and [M-H]⁻ refer to mono-isotopic molecular weights. The Biotage Optimizer™ microwave synthesizer is used in the standard configuration as delivered.

**TABLE 1**

| Ex. | **T**¹ | **T**² | **T**³ | MH + |
|---|---|---|---|---|
| 1 | 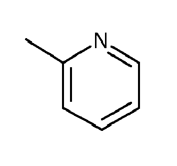 | 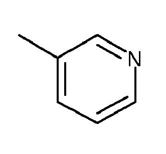 | 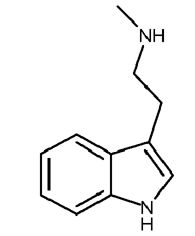 | 393 |
| 2 | 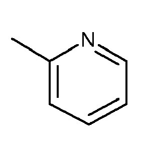 | 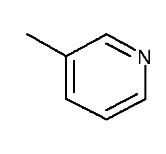 | 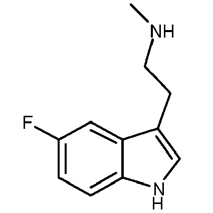 | 411 |
| 3 | 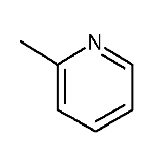 | 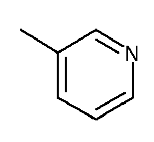 | 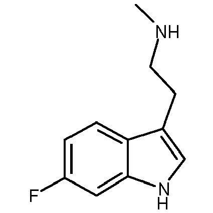 | 411 |
| 4 | 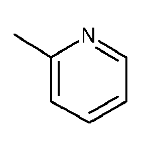 | 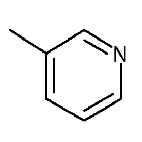 | 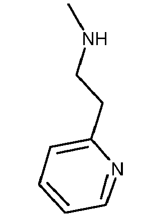 | 355 |
| 5 | 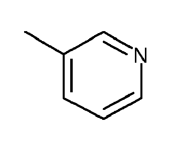 | 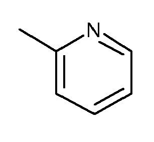 | 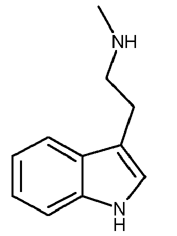 | 393 |
| 6 | 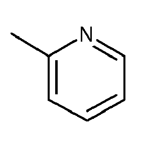 | 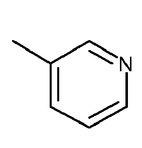 | 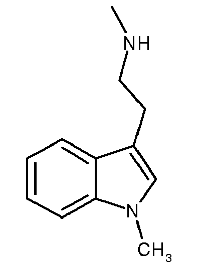 | 407 |
| 7 | 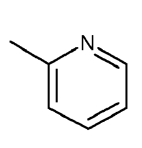 | 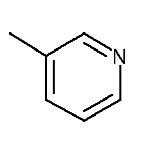 | 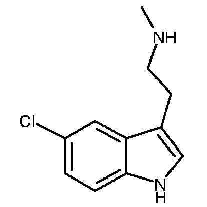 | 427 / 429 |
| 8 | 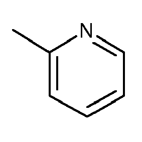 | 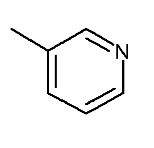 | 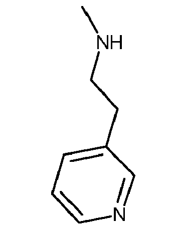 | 355 |
| 9 | 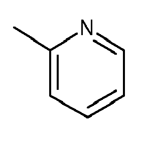 | 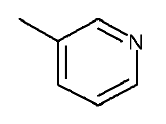 | 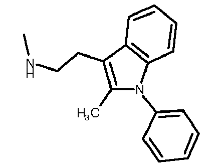 | - |
| 10 | 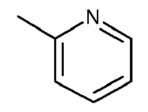 | 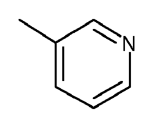 | 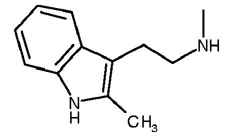 | - |
| 11 | 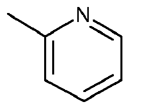 | 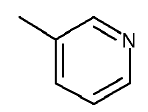 | 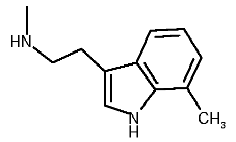 | 407 |
| 12 | 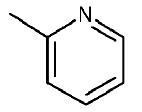 | 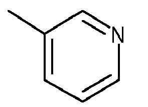 | 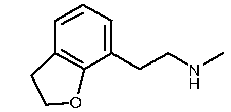 | - |
| 13 | 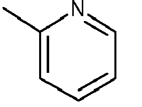 | 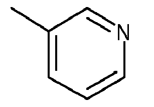 | 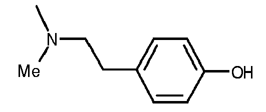 | 384 |
| 14 | 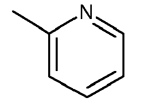 | 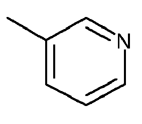 | 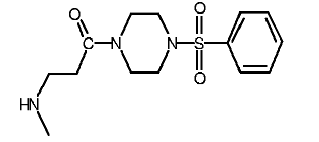 | - |
| 15 | 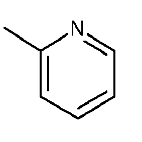 | 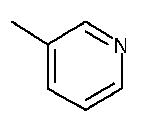 | 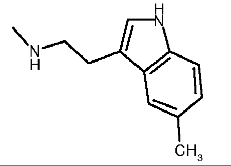 | 407 |
| 16 | 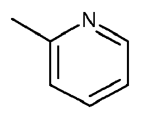 | 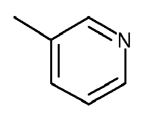 | 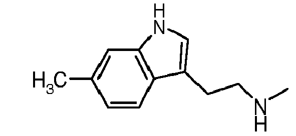 | 407 |
| 17 | 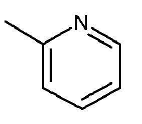 | 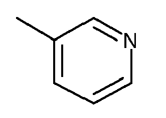 | 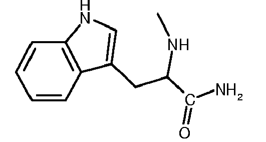 | 436 |
| 18 | 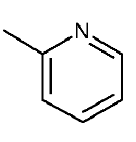 | 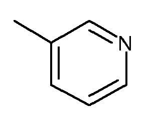 | 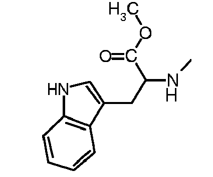 | 451 |
| 19 | 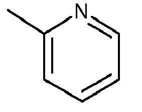 | 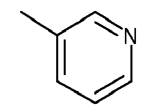 | 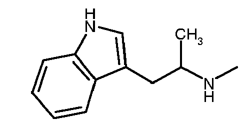 | 407 |
| 20 | 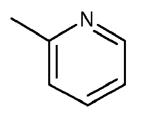 | 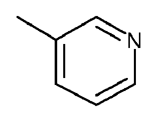 | 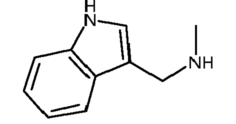 | - |
| 21 | 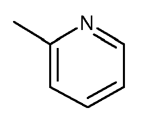 | 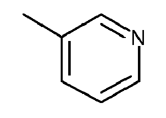 | 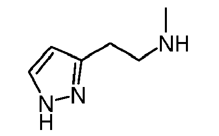 | - |
| 22 | 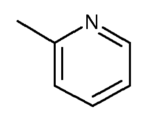 | 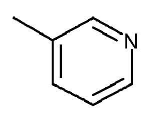 | 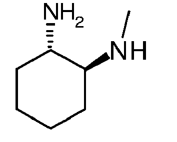 | 347 |
| 23 | 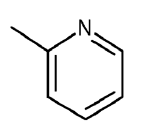 | 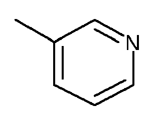 | 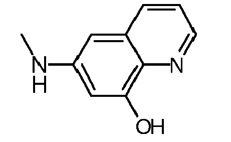 | 393 |
| 24 | 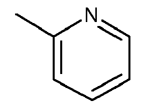 | 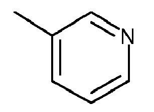 | 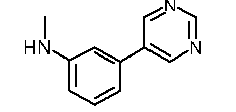 | 404 |
| 25 | 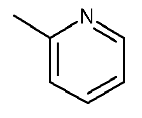 | 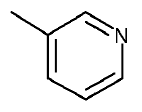 | 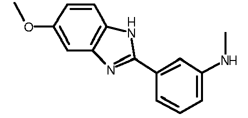 | - |
| 26 | 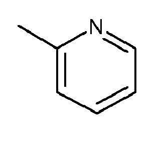 | 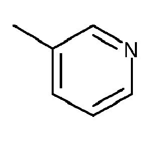 | 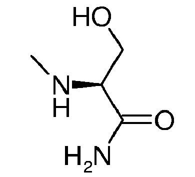 | 337 |
| 27 | 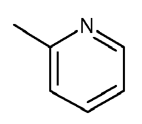 | 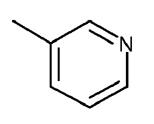 | 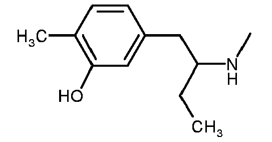 | 412 |
| 28 | 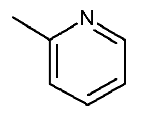 | 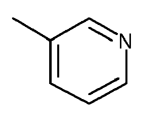 | 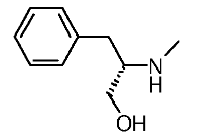 | 384 |
| 29 | 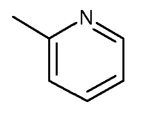 | 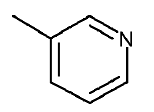 | 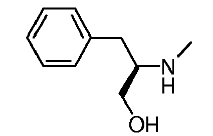 | 384 |
| 30 | 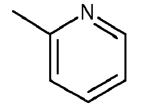 | 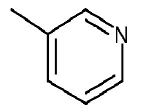 | 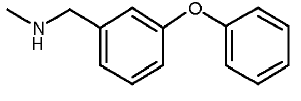 | - |
| 31 | 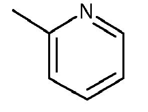 | 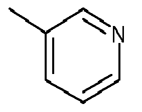 | 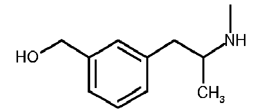 | - |
| 32 | 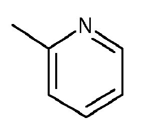 | 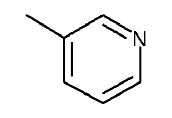 | 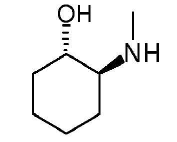 | 348 |
| 33 | 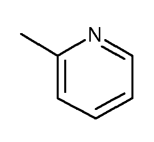 | 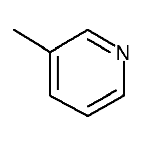 | 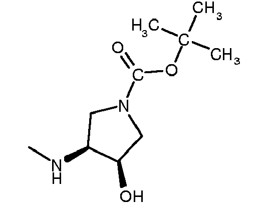 | - |
| 34 | 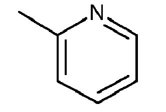 | 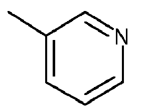 | 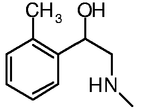 | - |
| 35 | 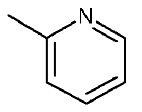 | 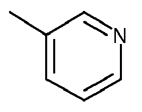 | 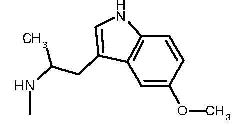 | - |
| 36 | 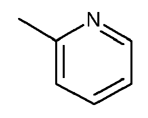 | 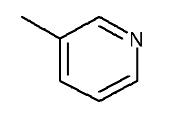 | 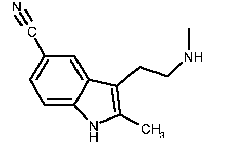 | - |
| 37 | 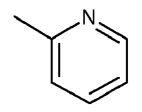 | 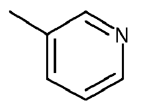 | 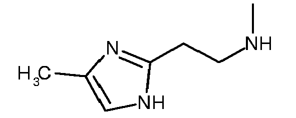 | - |
| 38 | 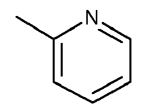 | 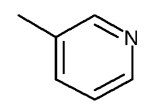 | 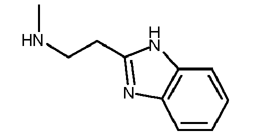 | - |
| 39 | 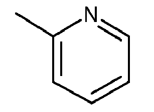 | 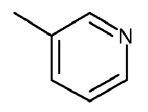 | 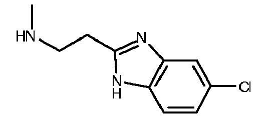 | - |
| 40 | 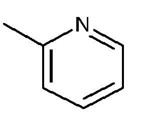 | 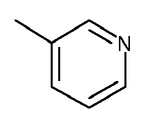 | 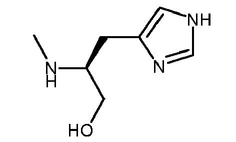 | 374 |
| 41 | 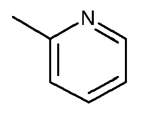 | 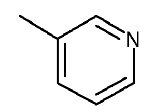 | 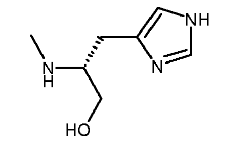 | - |
| 42 | 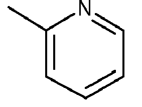 | 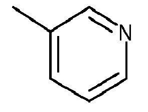 | 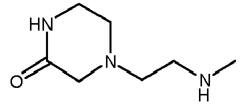 | - |
| 43 | 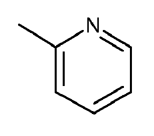 | 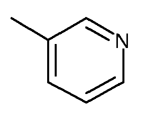 | 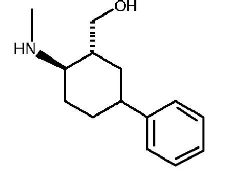 | - |
| 44 | 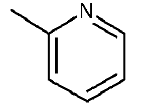 | 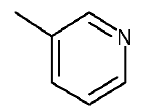 | 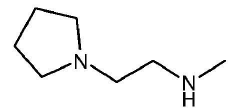 | 347 |
| 45 | 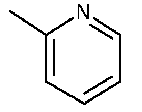 | 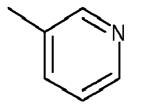 | 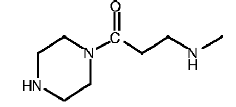 | - |
| 46 | 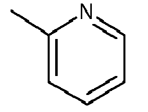 | 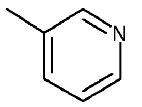 | 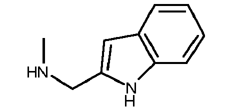 | - |
| 47 | 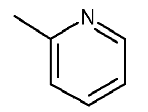 | 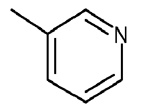 | 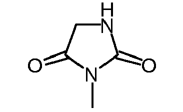 | - |
| 48 | 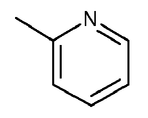 | 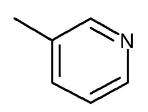 | 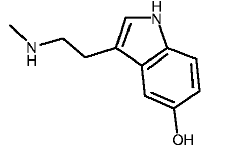 | 409 |
| 49 | 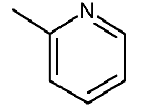 | 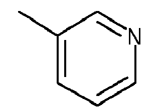 | 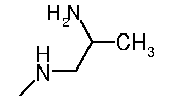 | 307 |
| 50 | 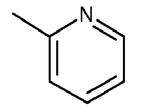 | 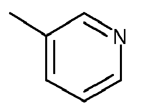 | 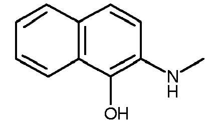 | - |
| 51 | 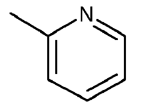 | 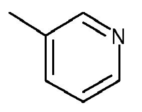 | 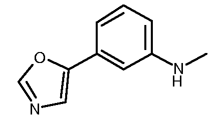 | 393 |
| 52 | 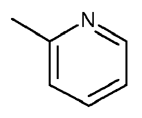 | 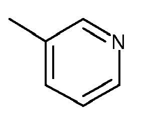 | 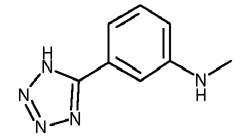 | 394 |
| 53 | 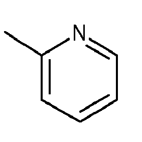 | 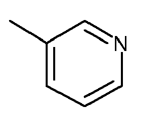 | 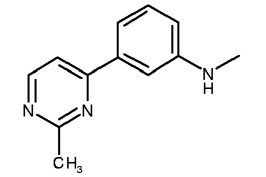 | 418 |
| 54 | 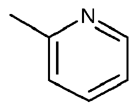 | 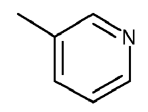 | 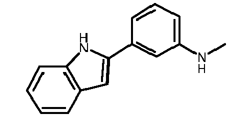 | - |
| 55 | 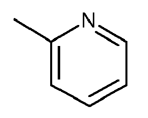 | 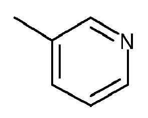 | 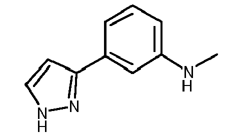 | - |
| 56 | 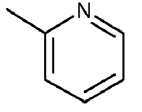 | 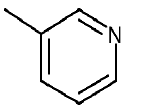 | 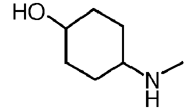 | 348 |
| 57 | 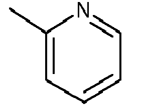 | 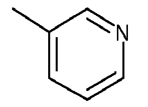 | 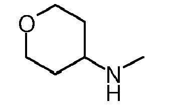 | 334 |
| 58 | 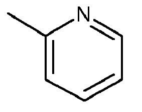 | 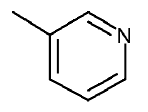 | 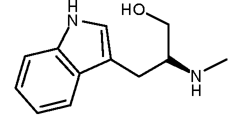 | 407 |
| 59 | 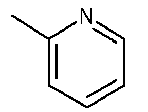 | 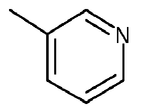 | 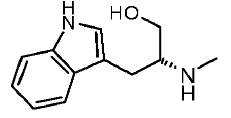 | - |
| 60 | 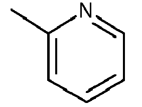 | 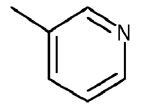 | 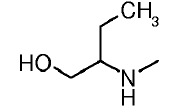 | 322 |
| 61 | 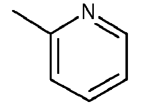 | 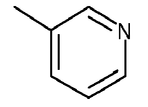 | 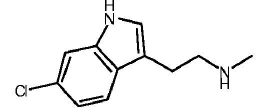 | - |
| 62 | 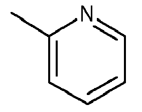 | 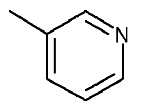 | 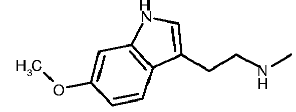 | - |
| 63 | 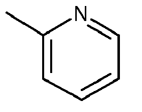 | 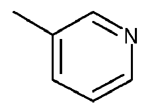 | 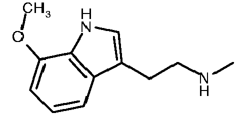 | - |
| 64 | 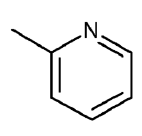 | 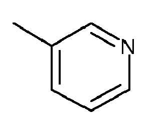 | 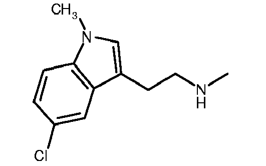 | - |
| 65 | 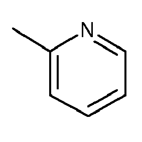 | 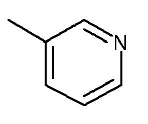 | 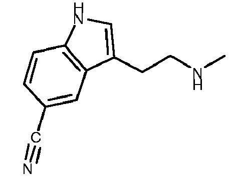 | - |
| 66 | 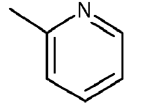 | 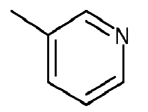 | 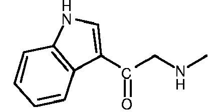 | - |
| 67 | 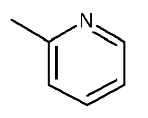 | 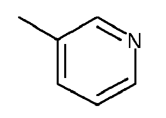 | 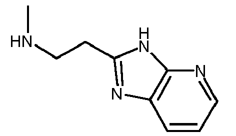 | - |
| 68 | 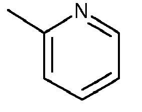 | 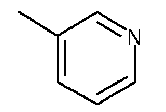 | 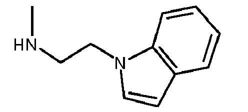 | - |
| 69 | 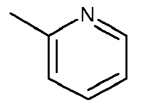 | 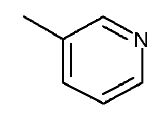 | 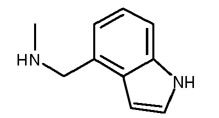 | 379 |
| 70 | 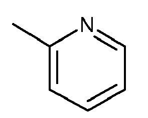 | 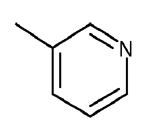 | 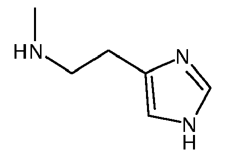 | 344 |
| 71 | 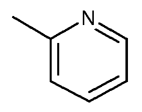 | 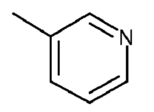 | 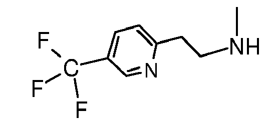 | - |
| 72 | 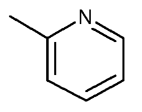 | 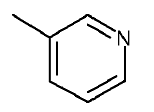 | 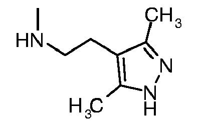 | 372 |
| 73 | 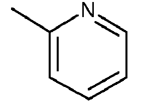 | 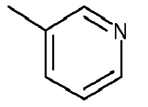 | 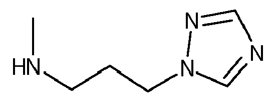 | 359 |
| 74 | 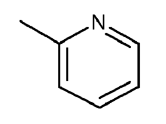 | 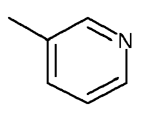 | 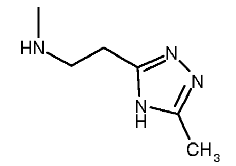 | - |
| 75 | 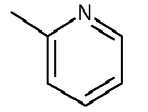 | 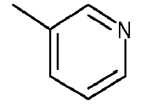 | 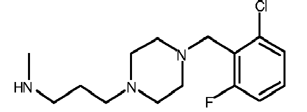 | - |
| 76 | 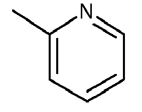 | 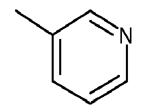 | 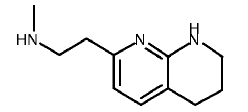 | - |
| 77 | 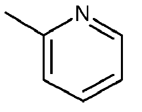 | 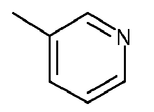 | 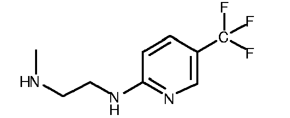 | - |
| 78 | 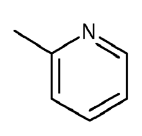 | 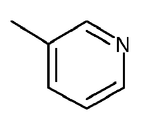 | 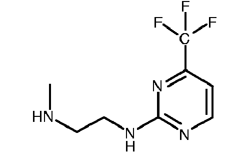 | - |
| 79 | 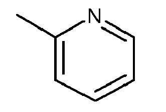 | 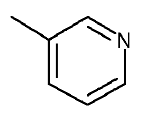 | 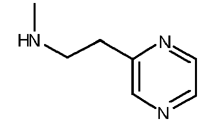 | - |
| 80 | 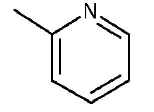 | 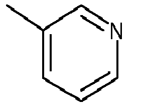 | 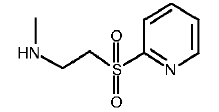 | - |
| 81 | 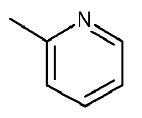 | 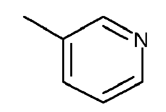 | 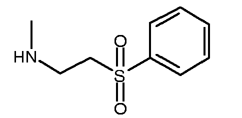 | - |
| 82 | 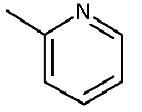 | 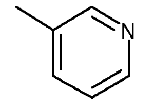 | 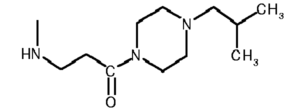 | - |
| 83 | 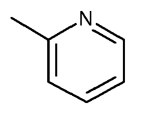 | 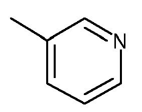 | 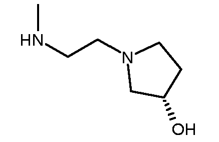 | - |
| 84 | 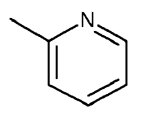 | 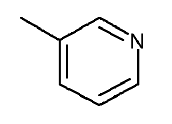 | 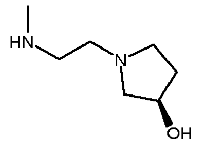 | - |
| 85 | 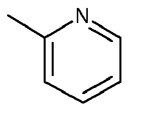 | 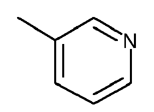 | 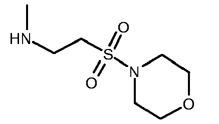 | - |
| 86 | 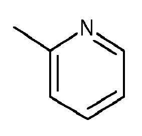 | 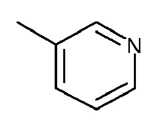 | 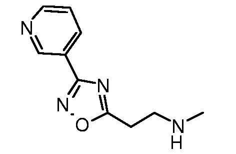 | - |
| 87 | 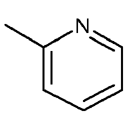 | 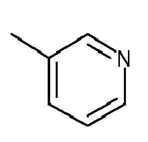 | 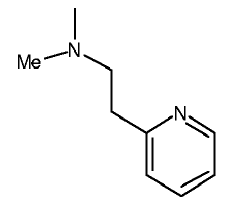 | 369 |
| 88 | 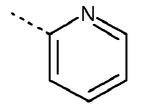 | 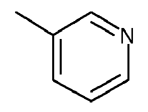 | 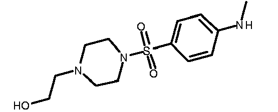 | - |
| 89 | 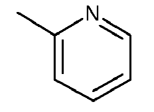 | 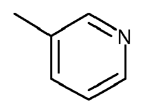 | 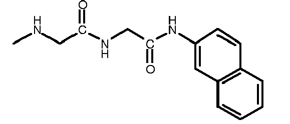 | 490 |
| 90 | 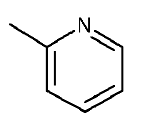 | 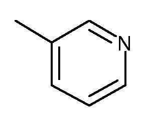 | 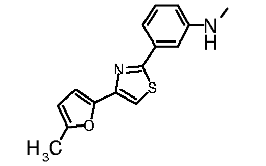 | - |
| 91 | 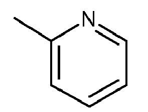 | 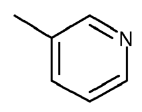 | 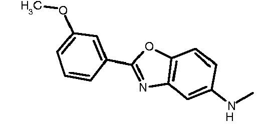 | - |
| 92 | 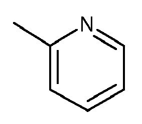 | 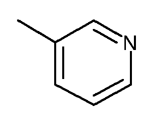 | 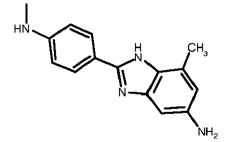 | - |
| 93 | 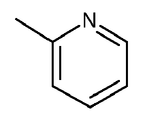 | 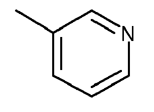 | 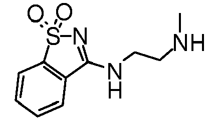 | - |
| 94 | 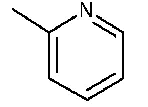 | 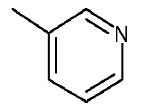 | 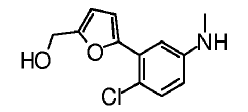 | - |
| 95 | 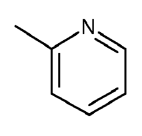 | 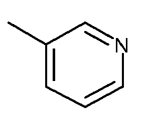 | 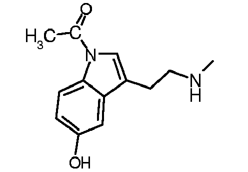 | - |
| 96 | 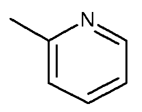 | 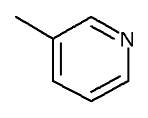 | 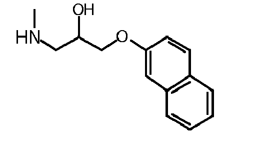 | - |
| 97 | 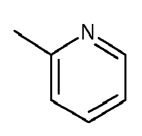 | 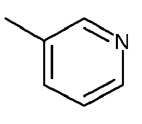 | 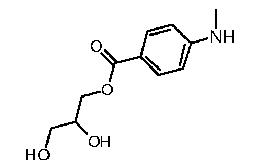 | - |
| 98 | 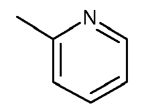 | 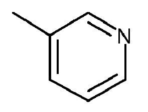 | 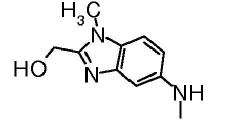 | - |
| 99 | 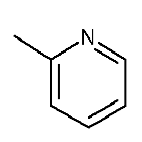 | 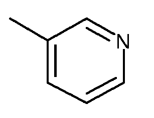 | 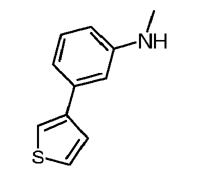 | - |
| 100 | 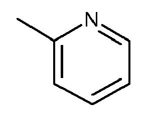 | 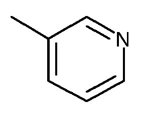 | 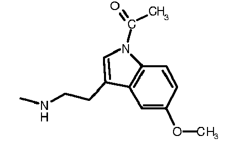 | - |
| 101 | 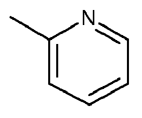 | 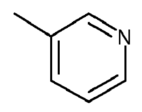 | 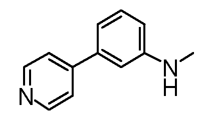 | - |
| 102 | 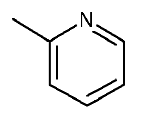 | 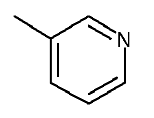 | 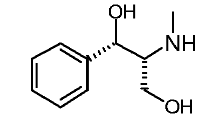 | 400 |
| 103 | 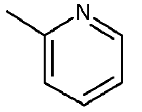 | 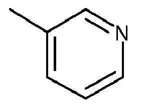 | 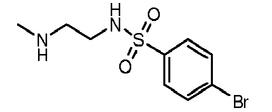 | - |
| 104 | 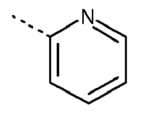 | 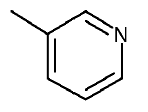 | 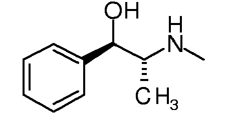 | - |
| 105 | 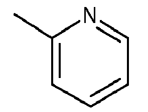 | 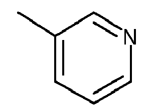 | 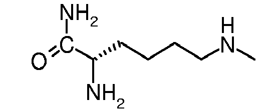 | - |
| 106 | 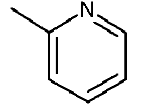 | 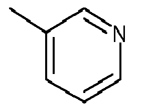 | 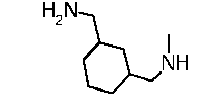 | 375 |
| 107 | 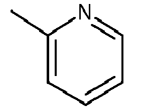 | 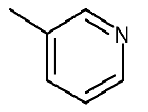 | 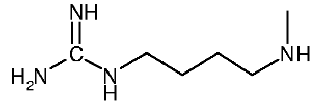 | - |
| 108 | 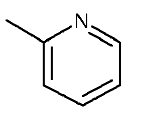 | 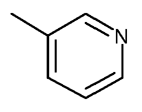 | 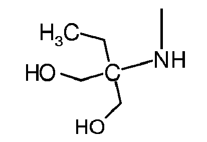 | 352 |
| 109 | 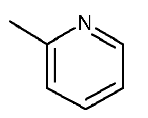 | 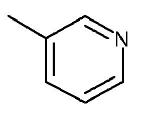 | 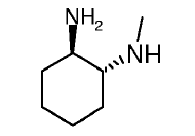 | 347 |
| 110 | 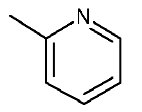 | 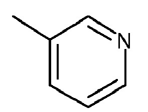 | 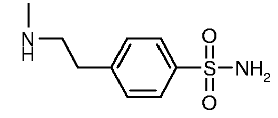 | 433 |
| 111 | 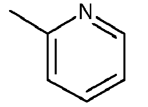 | 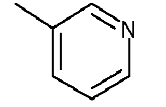 | 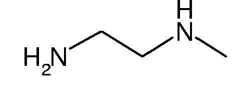 | 293 |
| 112 | 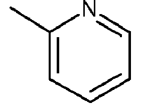 | 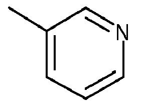 | 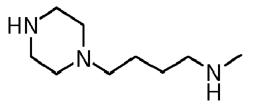 | - |
| 113 | 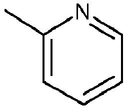 | 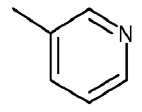 | 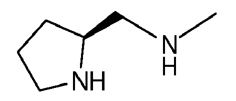 | - |
| 114 | 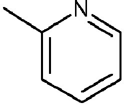 | 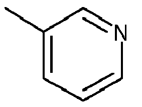 | 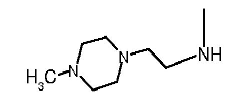 | 376 |
| 115 | 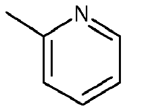 | 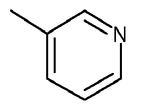 | 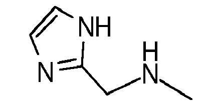 | - |
| 116 | 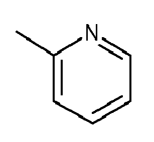 | 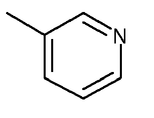 | 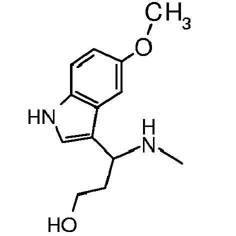 | - |
| 117 | 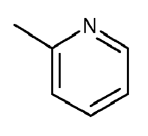 | 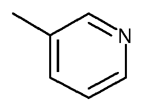 | 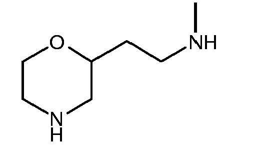 | - |
| 118 | 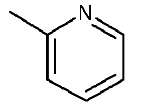 | 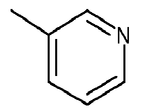 | 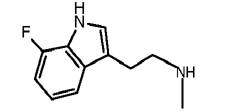 | - |
| 119 | 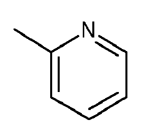 | 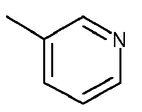 | 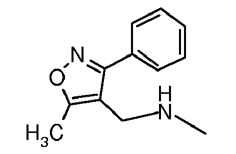 | 421 |
| 120 | 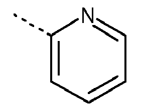 | 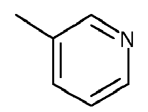 | 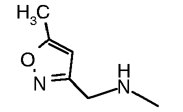 | - |
| 121 | 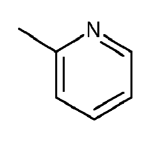 | 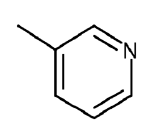 | 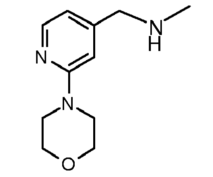 | - |
| 122 | 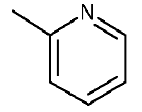 | 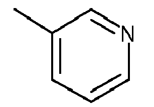 | 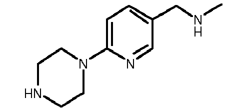 | - |
| 123 | 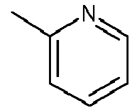 | 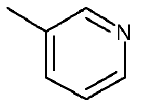 | 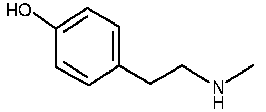 | 370 |
| 124 | 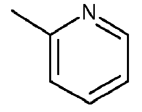 | 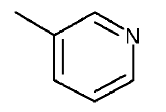 | 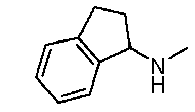 | 366 |
| 125 | 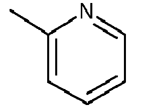 | 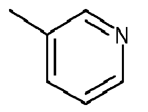 | 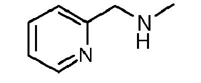 | 341 |
| 126 | 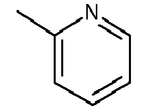 | 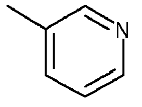 | 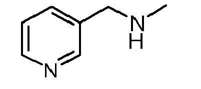 | 341 |
| 127 | 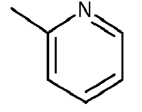 | 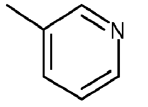 | 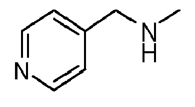 | 341 |
| 128 | 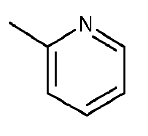 | 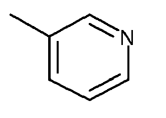 | 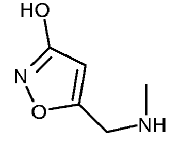 | - |
| 129 | 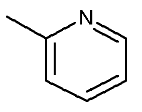 | 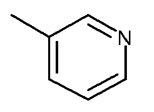 | 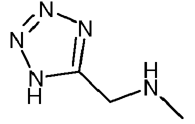 | - |
| 130 | 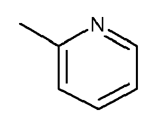 | 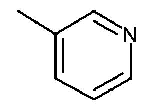 | 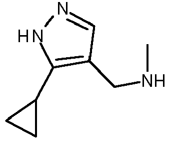 | - |
| 131 | 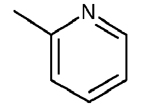 | 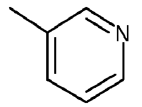 | 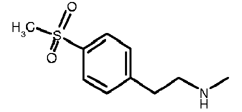 | - |
| 132 | 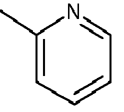 | 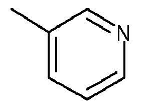 | 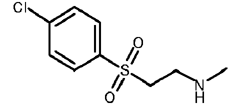 | 432 |
| 133 | 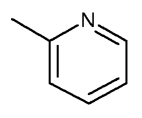 | 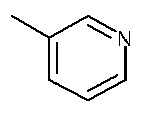 | 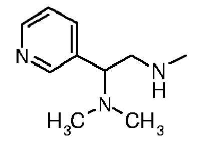 | - |
| 134 | 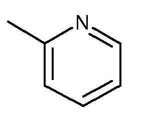 | 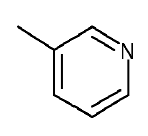 | 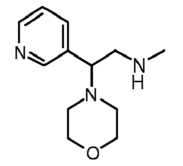 | - |
| 135 | 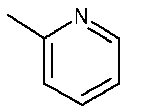 | 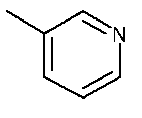 | 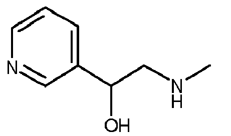 | - |
| 136 | 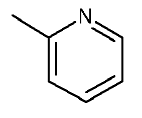 | 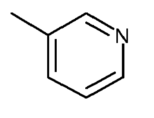 | 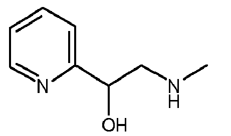 | - |
| 137 | 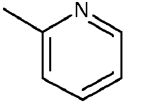 | 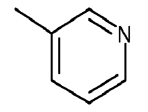 | 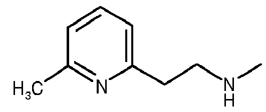 | - |
| 138 | 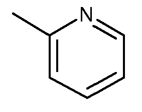 | 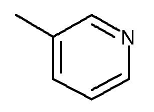 | 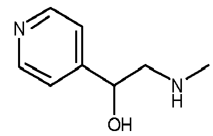 | - |
| 139 | 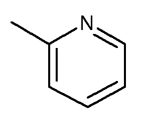 | 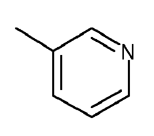 | 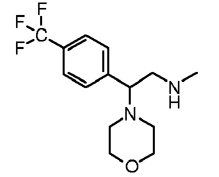 | - |
| 140 | 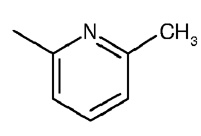 | 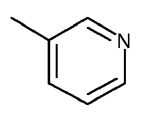 | 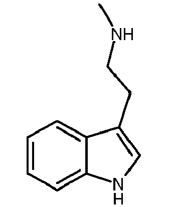 | 407 |
| 141 | 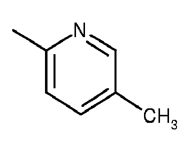 | 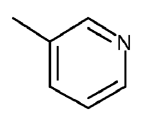 | 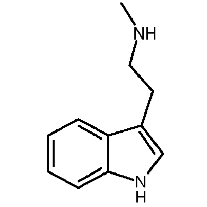 | - |
| 142 | 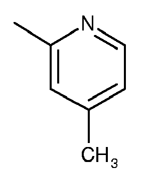 | 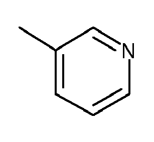 | 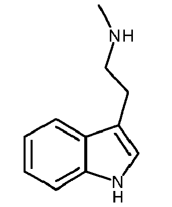 | - |
| 143 | 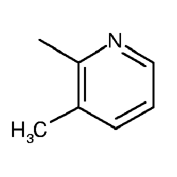 | 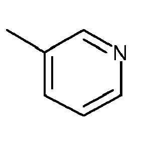 | 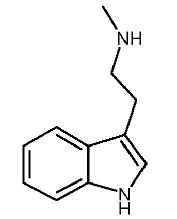 | - |
| 144 | 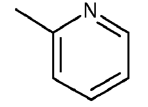 | 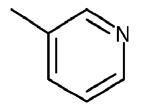 | -NH₂ | 250 |
| 145 | 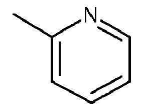 | 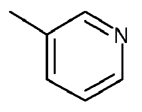 | 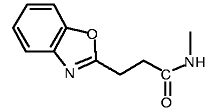 | - |
| 146 | 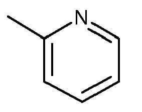 | 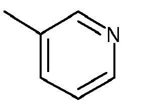 | 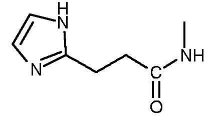 | - |
| 147 | 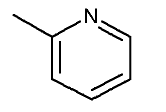 | 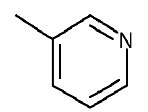 | 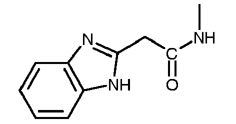 | - |
| 148 | 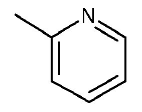 | 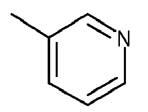 | 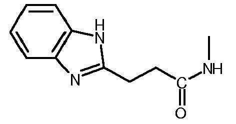 | - |
| 149 | 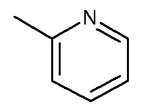 | 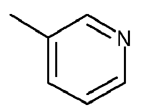 | 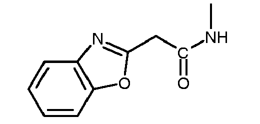 | - |
| 150 | 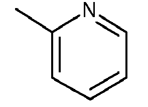 | 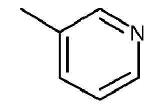 | 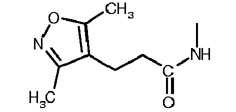 | - |
| 151 | 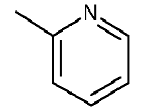 | 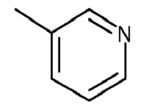 | 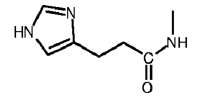 | - |
| 152 | 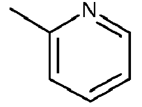 | 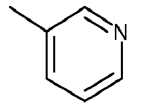 | 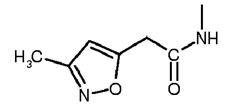 | - |
| 153 | 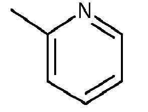 | 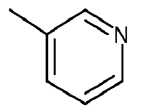 | 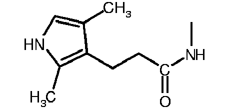 | - |
| 154 | 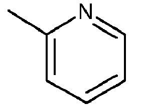 | 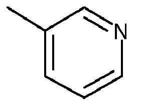 | 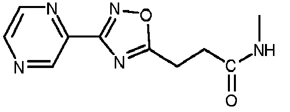 | - |
| 155 | 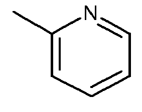 | 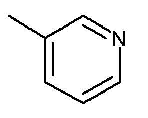 | 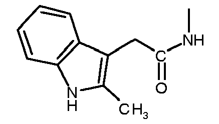 | - |
| 156 | 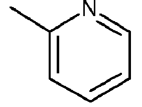 | 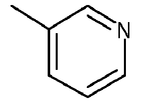 | 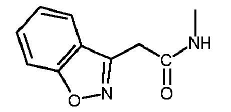 | - |
| 157 | 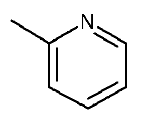 | 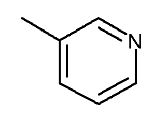 | 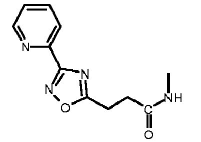 | - |
| 158 | 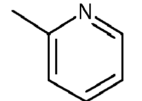 | 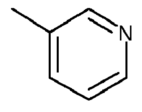 | 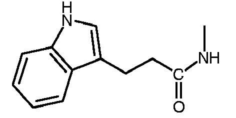 | - |
| 159 | 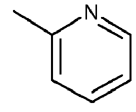 | 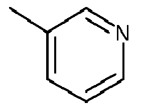 | 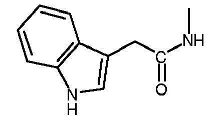 | - |
| 160 | 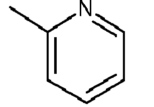 | 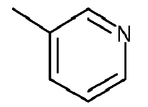 | 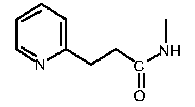 | - |
| 161 | 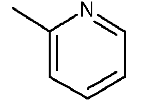 | 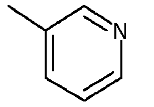 | 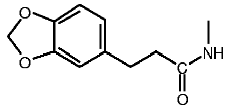 | - |
| 162 | 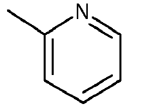 | 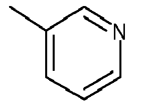 | 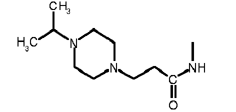 | - |
| 163 | 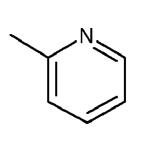 | 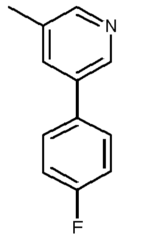 | 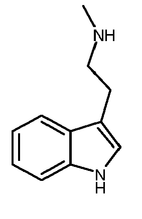 | - |

### Abbreviations

DCM is dichloromethane, DMF is dimethylformamide, DIPEA is N,N-diisopropylethylamine, Et₃N is triethylamine, EtOAc is ethyl acetate, EtOH is ethanol, H₂O is water, HATU is O-(7-azabenzotriazol-1-yl)-N, N, N', N'-tetramethyluronium hexafluorophosphate, HPLC is high performance liquid chromatography, MgSO₄ is magnesium sulfate, NMP is N - methylpyrrol-idinone, MeOH is methanol, NaOH is sodium hydroxide, ,Pd is palladium, K₂CO₃ is potassium carbonate, NH₃(g) is ammonia (gas), and RT is room temperature.

### Preparation of intermediate compounds

### 4-Chloro-6-pyridin-3-yl-2-pyridin-2-yl-pyrimidine

To 6-pyridin-3-yl-2-pyridin-2-yl-3H-pyrimidin-4-one (1eq, 0.26 mmol, 0.035 g) under inert atmosphere, is added dropwise phosphorus oxychloride (15 eq, 3.9mmol, 0.36 ml) followed by cautious addition of phosphorus pentachloride (1eq, 0.26 mmol, 0.054 g). After 4 hours at reflux, the reaction mixture is added slowly to ice/water. The mixture is evaporated under reduced pressure to give a solid residue. The crude product is purified by flash chromatography (DCM / MeOH 100:0 to 0:100) to afford 4-chloro-6-pyridin-3-yl-2-pyridin-2-yl-pyrimidine as a white solid; MH+ = 269/271

### 6-Pyridin-3-yl-2-pyridin-2-yl-pyrimidin-4-ol

To a solution of pyridine-2-carboxamide (1.2 eq, 3.4 mmol, 0.41 g) in H₂O (1.4 ml) and NaOH (1.5 eq, 4.3 mmol, 0.17 g), is added a solution of 3-oxo-3-pyridin-3-yl-propionic acid ethyl ester (1eq, 2.85 mmol, 0.55 g) in EtOH (1.7 ml) at room temperature. The reaction is left to stir for 15 hours. The reaction solvent was removed under reduced pressure to give a solid residue. The crude product is purified by flash chromatography (DCM / MeOH 100:0 to 0:100) to afford 6-pyridin-3-yl-2-pyridin-2-yl-3H-pyrimidin-4-one as a white solid; MH+= 251

### [6-(5-Bromo-pyridin-3-yl)-2-pyridin-2-yl-pyrimidin-4-yl]-[2-(1H-indol-3-yl)-ethyl]-amine

[6-(5-Bromo-pyridin-3-yl)-2-pyridin-2-yl-pyrimidin-4-yl]-[2-(1H-indol-3-yl)-ethyl]-amine is prepared analogously to [2-(1H-Indol-3-yl)-ethyl]-(6-pyridin-3-yl-2-pyridin-2-yl-pyrimidin-4-yl)-amine by replacing 3-oxo-3-pyridin-3-yl-propionic acid ethyl ester with 3-(5-bromo-pyridin-3-yl)-3-oxo-propionic acid ethyl ester.

### N-Hydroxy-6-methyl-pyridine-2-carboxamidine

To a solution of 6-methyl-2-pyridnecarbonitrile (1 eq, 4.23 mmol, 0.50 g) in EtOH (6 ml) is added a solution of hydroxylamine chloride (3.6 eq, 15.2 mmol, 1.06 g) in H₂O (3 ml) and sodium carbonate (3.2 eq, 13.5 mmol, 1.44 g) in H₂O (3 ml) at room temperature. The reaction mixture is heated at reflux for 15 hours. The reaction mixture is cooled to room temperature and filtered, rinsing the solid with H₂O. The residue is dried under vacuum to afford N-Hydroxy-6-methyl-pyridine-2-carboxamidine, as a white solid; MH+ 152

### 6-Methyl-pyridine-2-carboxamidine

To a solution of N-hydroxy-6-methyl-pyridine-2-carboxamidine (1 eq, 2.68 mmol, 0.40 g) in acetic acid (5 ml) at room temperature, under argon, is added ammonium formate (5 eq, 13.4mmol, 0.844g) and Pd on carbon (10% activated, 0.2 eq, 0.54 mmol, 0.057 g). The reaction mixture is heated at reflux for 6 hours. The reaction mixture is cooled to room temperature, and the reaction solvent is removed under reduced pressure to give a solid residue. The crude product is purified by flash chromatography (DCM : MeOH; 1:1) . The solvent is removed under reduced pressure and the residue is azeotroped with iso-hexane. The residue is dried under vacuum to afford 6-methyl-pyridine-2-carboxamidine, as an off-white solid; 2M+ = 271.

Other amidine intermediates can be prepared using the method described in R Fuks, Tetrahedron, 1973, 29, 2147-2151.

### Preparation of final compounds

### Example 1

### [2-(1H-Indol-3-yl)-ethyl]-(6-pyridin-3-yl-2-pyridin-2-yl-pyrimidin-4-yl)-amine

To a solution of 4-chloro-6-pyridin-3-yl-2-pyridin-2-yl-pyrimidine (1eq, 30 mg, 0.11 mmol) in NMP (0.8mL) and K₂C0₃ (1.1eq, 0.12mmol, 16.7mg) is added tryptamine (1eq, 0.11 mmol, 17.6mg). The reaction is heated in the microwave at 120°C for 90 minutes. The reaction mixture is purified by flash chromatography to afford [2-(1H-indol-3-yl)-ethyl]-(6-pyridin-3-yl-2-pyridin-2-yl-pyrimidin-4-yl)-amine; MH+ 393

### Examples 2 to 143

These examples namely,
[2-(5-Fluoro-1H-indol-3-yl)-ethyl]-(6-pyridin-3-yl-2-pyridin-2-yl-pyrimidin-4-yl)-amine (Ex. 2),
[2-(6-Fluoro-1H-indol-3-yl)-ethyl]-(6-pyridin-3-yl-2-pyridin-2-yl-pyrimidin-4-yl)-amine (Ex. 3),
(2-Pyridin-2-yl-ethyl)-(6-pyridin-3-yl-2-pyridin-2-yl-pyrimidin-4-yl)-amine (Ex. 4),
[2-(1H-Indol-3-yl)-ethyl]-(2-pyridin-3 -yl-6-pyridin-2-yl-pyrimidin-4-yl)-amine (Ex. 5),
[2-(1-Methyl-1H-indol-3-yl)-ethyl]-(6-pyridin-3-yl-2-pyridin-2-yl-pyrimidin-4-yl)-amine (Ex. 6),
[2-(5-Chloro-1H-indol-3-yl)-ethyl]-(6-pyridin-3-yl-2-pyridin-2-yl-pyrimidin-4-yl)-amine (Ex. 7),
(2-Pyridin-3-yl-ethyl)-(6-pyridin-3-yl-2-pyridin-2-yl-pyrimidin-4-yl)-amine (Ex. 8),
[2-(2-Methyl-1-phenyl-1H -indol-3-yl)-ethyl]-(6-pyridin-3-yl-2-pyridin-2-yl-pyrimidin-4-yl)-amine (Ex. 9),
[2-(2-Methyl-1H-indol-3-yl)-ethyl]-(6-pyridin-3-yl-2-pyridin-2-yl-pyrimidin-4-yl)-amine (Ex. 10),
[2-(7-Methyl-1H-indol-3-yl)-ethyl]-(6-pyridin-3-yl-2-pyridin-2-yl-pyrimidin-4-yl)-amine (Ex. 11),
[2-(2,3-Dihydro-benzofuran-7-yl)-ethyl]-(6-pyridin-3-yl-2-pyridin-2-yl-pyrimidin-4-yl)-amine (Ex. 12),
4-{2-[Methyl-(6-pyridin-3-yl-2-pyridin-2-yl-pyrimidin-4-yl)-amino]-ethyl}-phenol (Ex. 13),
1-(4-Benzenesulfonyl-piperazin-1-yl)-3-(6-pyridin-3-yl-2-pyridin-2-yl-pyrimidin-4-ylamino)-propan-1-one (Ex. 14),
[2-(5-Methyl-1H-indol-3-yl)-ethyl]-(6-pyridin-3-yl-2-pyridin-2-yl-pyrimidin-4-yl)-amine (Ex. 15),
[2-(6-Methyl-1H-indol-3-yl)-ethyl]-(6-pyridin-3-yl-2-pyridin-2-yl-pyrimidin-4-yl)-amine (Ex. 16),
3-(1H-Indol-3-yl)-2-(6-pyridin-3-yl-2-pyridin-2-yl-pyrimidin-4-ylamino)-propionamide (Ex. 17),
3-(1H-Indol-3-yl)-2-(6-pyridin-3-yl-2-pyridin-2-yl-pyrimidin-4-ylamino)-propionic acid methyl ester (Ex. 18),
[2-(1H-Indol-3-yl)-1-methyl-ethyl]-(6-pyridin-3-yl-2-pyridin-2-yl-pyrimidin-4-yl)-amine (Ex. 19),
(1H-Indol-3-ylmethyl)-(6-pyridin-3-yl-2-pyridin-2-yl-pyrimidin-4-yl)-amine (Ex. 20),
[2-(1H-Pyrazol-3-yl)-ethyl]-(6-pyridin-3-yl-2-pyridin-2-yl-pyrimidin-4-yl)-amine (Ex. 21),
(1S,2S)-N-(6-Pyridin-3-yl-2-pyridin-2-yl-pyrimidin-4-yl)-cyclohexane-1,2-diamine (Ex. 22),
6-(6-Pyridin-3-yl-2-pyridin-2-yl-pyrimidin-4-ylamino)-quinolin-8-ol (Ex. 23),
(6-Pyridin-3-yl-2-pyridin-2-yl-pyrimidin-4-yl)-(3-pyrimidin-5-yl-phenyl)-amine (Ex. 24),
[3-(5-Methoxy-1H-benzoimidazol-2-yl)-phenyl]-(6-pyridin-3-yl-2-pyridin-2-yl-pyrimidin-4-yl)-amine (Ex. 25),
(S)-3-Hydroxy-2-(6-pyridin-3-yl-2-pyridin-2-yl-pyrimidin-4-ylamino)-propionamide (Ex. 26),
2-Methyl-5-[2-(6-pyridin-3-yl-2-pyridin-2-yl-pyrimidin-4-ylamino)-butyl]-phenol (Ex. 27),
(S)-3-Phenyl-2-(6-pyridin-3-yl-2-pyridin-2-yl-pyrimidin-4-ylamino)-propan-1-ol (Ex. 28),
(R)-3-Phenyl-2-(6-pyridin-3-yl-2-pyridin-2-yl-pyrimidin-4-ylamino)-propan-1-ol (Ex. 29),
(3-Phenoxy-benzyl)-(6-pyridin-3-yl-2-pyridin-2-yl-pyrimidin-4-yl)-amine (Ex. 30),
{3-[2-(6-Pyridin-3-yl-2-pyridin-2-yl-pyrimidin-4-ylamino)-propyl]-phenyl}-methanol (Ex. 31),
(1 S,2S)-2-(6-Pyridin-3-yl-2-pyridin-2-yl-pyrimidin-4-ylamino)-cyclohexanol (Ex. 32),
(3R,4S)-3-Hydroxy-4-(6-pyridin-3-yl-2-pyridin-2-yl-pyrimidin-4-ylamino)-pyrrolidine-1-carboxylic acid tert-butyl ester (Ex. 33),
2-(6-Pyridin-3-yl-2-pyridin-2-yl-pyrimidin-4-ylamino)-1-o-tolyl-ethanol (Ex. 34),
[2-(5-Methoxy-1H-indol-3-yl)-1-methyl-ethyl]-(6-pyridin-3-yl-2-pyridin-2-yl-pyrimidin-4-yl)-amine (Ex. 35),
2-Methyl-3-[2-(6-pyridin-3-yl-2-pyridin-2-yl-pyrimidin-4-ylamino)-ethyl]-1H-indole-5-carbonitrile (Ex. 36),
[2-(4-M ethyl-1H -imidazol-2-yl)-ethyl]-(6-pyridin-3-yl-2-pyridin-2-yl-pyrimidin-4-yl)-amine (Ex. 37),
[2-(1H-Benzoimidazol-2-yl)-ethyl]-(6-pyridin-3-yl-2-pyridin-2-yl-pyrimidin-4-yl)-amine (Ex. 38),
[2-(5-Chloro-1H -benzoimidazol-2-yl)-ethyl]-(6-pyridin-3-yl-2-pyridin-2-yl-pyrimidin-4-yl)-amine (Ex. 39),
(S)-3-(1H-Imidazol-4-yl)-2-(6-pyridin-3-yl-2-pyridin-2-yl-pyrimidin-4-ylamino)-propan-1-ol (Ex. 40),
(R)-3-(1 H -Imidazol-4-yl)-2-(6-pyridin-3-yl-2-pyridin-2-yl-pyrimidin-4-ylamino)-propan-1-ol (Ex. 41),
4-[2-(6-Pyridin-3-yl-2-pyridin-2-yl-pyrimidin-4-ylamino)-ethyl]-piperazin-2-one (Ex. 42),
[(1R,2R,5S)-5-Phenyl-2-(6-pyridin-3-yl-2-pyridin-2-yl-pyrimidin-4-ylamino)-cyclohexyl]-methanol (Ex. 43),
(6-Pyridin-3-yl-2-pyridin-2-yl-pyrimidin-4-yl)-(2-pyrrolidin-1-yl-ethyl)-amine (Ex. 44),
1-Piperazin-1-yl-3-(6-pyridin-3-yl-2-pyridin-2-yl-pyrimidin-4-ylamino)-propan-1-one (Ex. 45),
(1H-Indol-2-ylmethyl)-(6-pyridin-3-yl-2-pyridin-2-yl-pyrimidin-4-yl)-amine (Ex. 46),
3-(6-Pyridin-3-yl-2-pyridin-2-yl-pyrimidin-4-yl)-imidazolidine-2,4-dione (Ex. 47),
3-[2-(6-Pyridin-3-yl-2-pyridin-2-yl-pyrimidin-4-ylamino)-ethyl]-1H-indol-5-ol (Ex. 48),
N* 1 *-(6-Pyridin-3-yl-2-pyridin-2-yl-pyrimidin-4-yl)-propane-1,2-diamine (Ex. 49),
2-(6-Pyridin-3-yl-2-pyridin-2-yl-pyrimidin-4-ylamino)-naphthalen-1-ol (Ex. 50),
(3-Oxazol-5-yl-phenyl)-(6-pyridin-3-yl-2-pyridin-2-yl-pyrimidin-4-yl)-amine (Ex. 51),
(6-Pyridin-3-yl-2-pyridin-2-yl-pyrimidin-4-yl)-[3-(1H-tetrazol-5-yl)-phenyl]-amine (Ex. 52),
[3-(2-Methyl-pyrimidin-4-yl)-phenyl]-(6-pyridin-3-yl-2-pyridin-2-yl-pyrimidin-4-yl)-amine (Ex. 53),
[3-(1H-Indol-2-yl)-phenyl]-(6-pyridin-3-yl-2-pyridin-2-yl-pyrimidin-4-yl)-amine (Ex. 54),
[3-(1H-Pyrazol-3-yl)-phenyl]-(6-pyridin-3-yl-2-pyridin-2-yl-pyrimidin-4-yl)-amine (Ex. 55),
4-(6-Pyridin-3-yl-2-pyridin-2-yl-pyrimidin-4-ylamino)-cyclohexanol (Ex. 56),
(6-Pyridin-3-yl-2-pyridin-2-yl-pyrimidin-4-yl)-(tetrahydro-pyran-4-yl)-amine (Ex. 57),
(S)-3-(1H-Indol-3-yl)-2-(6-pyridin-3-yl-2-pyridin-2-yl-pyrimidin-4-ylamino)-propan-1-ol (Ex. 58),
(R)-3-(1H-Indol-3-yl)-2-(6-pyridin-3-yl-2-pyridin-2-yl-pyrimidin-4-ylamino)-propan-1-ol (Ex. 59),
2-(6-Pyridin-3-yl-2-pyridin-2-yl-pyrimidin-4-ylamino)-butan-1-ol (Ex. 60),
[2-(6-Chloro-1H-indol-3-yl)-ethyl]-(6-pyridin-3-yl-2-pyridin-2-yl-pyrimidin-4-yl)-amine (Ex. 61),
[2-(6-Methoxy-1H-indol-3-yl)-ethyl]-(6-pyridin-3-yl-2-pyridin-2-yl-pyrimidin-4-yl)-amine (Ex. 62),
[2-(7-Methoxy-1H-indol-3-yl)-ethyl]-(6-pyridin-3-yl-2-pyridin-2-yl-pyrimidin-4-yl)-amine (Ex. 63),
[2-(5 -Chloro-1-methyl-1H -indol-3-yl)-ethyl]-(6-pyridin-3-yl-2-pyridin-2-yl-pyrimidin-4-yl)-amine (Ex. 64),
3-[2-(6-Pyridin-3 -yl-2-pyridin-2-yl-pyrimidin-4-ylamino)-ethyl]- 1H -indole-5-carbonitrile (Ex. 65),
1-(1H-Indol-3-yl)-2-(6-pyridin-3-yl-2-pyridin-2-yl-pyrimidin-4-ylamino)-ethanone (Ex. 66),
[2-(3H-Imidazo[4,5-b]pyridin-2-yl)-ethyl]-(6-pyridin-3-yl-2-pyridin-2-yl-pyrimidin-4-yl)-amine (Ex. 67),
(2-Indol-1-yl-ethyl)-(6-pyridin-3-yl-2-pyridin-2-yl-pyrimidin-4-yl)-amine (Ex. 68),
(1H-hidol-4-ylmethyl)-(6-pyridin-3-yl-2-pyridin-2-yl-pyrimidin-4-yl)-amine (Ex. 69),
[2-(1H-Imidazol-4-yl)-ethyl]-(6-pyridin-3-yl-2-pyridin-2-yl-pyrimidin-4-yl)-amine (Ex. 70),
(6-Pyridin-3-yl-2-pyridin-2-yl-pyrimidin-4-yl)-[2-(5-trifluoromethyl-pyridin-2-yl)-ethyl]-amine (Ex. 71),
[2-(3,5-Dimethyl-1H-pyrazol-4-yl)-ethyl]-(6-pyridin-3-yl-2-pyridin-2-yl-pyrimidin-4-yl)-amine (Ex. 72),
(6-Pyridin-3-yl-2-pyridin-2-yl-pyrimidin-4-yl)-(3-[1,2,4]triazol-1-yl-propyl)-amine (Ex. 73),
[2-(5-M ethyl-4H -[1,2,4]triazol-3-yl)-ethyl]-(6-pyridin-3-yl-2-pyridin-2-yl-pyrimidin-4-yl)-amine (Ex. 74),
{3-[4-(2-Chloro-6-fluoro-benzyl)-piperazin-1-yl]-propyl}-(6-pyridin-3-yl-2-pyridin-2-yl-pyrimidin-4-yl)-amine (Ex. 75),
(6-Pyridin-3-yl-2-pyridin-2-yl-pyrimidin-4-yl)-[2-(5,6,7,8-tetrahydro-[1,8]naphthyridin-2-yl)-ethyl]-amine (Ex. 76),
N -(2-Pyridin-2-yl-6-pyridin-3-yl-pyrimidin-4-yl)-N'-(5-trifluoromethyl-pyridin-2-yl)-ethane-1,2-diamine (Ex. 77),
N -(2-Pyridin-2-yl-6-pyridin-3-yl-pyrimidin-4-yl)-N' -(4-trifluoro methyl-pyrimidin-2-yl)-ethane-1,2-diamine (Ex. 78),
(2-Pyrazin-2-yl-ethyl)-(6-pyridin-3-yl-2-pyridin-2-yl-pyrimidin-4-yl)-amine (Ex. 79),
[2-(Pyridine-2-sulfonyl)-ethyl]-(6-pyridin-3-yl-2-pyridin-2-yl-pyrimidin-4-yl)-amine (Ex. 80),
(2-Benzenesulfonyl-ethyl)-(2-pyridin-2-yl-6-pyridin-3-yl-pyrimidin-4-yl)-amine (Ex. 81),
1-(4-Isobutyl-piperazin-1-yl)-3-(6-pyridin-3-yl-2-pyridin-2-yl-pyrimidin-4-ylamino)-propan-1-one (Ex. 82),
(S)-1-[2-(6-Pyridin-3-yl-2-pyridin-2-yl-pyrimidin-4-ylamino)-ethyl]-pyrrolidin-3-ol (Ex. 83),
(R)-1-[2-(6-Pyridin-3-yl-2-pyridin-2-yl-pyrimidin-4-ylamino)-ethyl]-pyrrolidin-3-ol (Ex. 84),
[2-(Morpholine-4-sulfonyl)-ethyl]-(6-pyridin-3-yl-2-pyridin-2-yl-pyrimidin-4-yl)-amine (Ex. 85),
[2-(3-Pyridin-3-yl-[1,2,4]oxadiazol-5-yl)-ethyl]-(6-pyridin-3-yl-2-pyridin-2-yl-pyrimidin-4-yl)-amine (Ex. 86),
Methyl-(2-pyridin-2-yl-ethyl)-(6-pyridin-3-yl-2-pyridin-2-yl-pyrimidin-4-yl)-amine (Ex. 87),
2-{4-[4-(6-Pyridin-3-yl-2-pyridin-2-yl-pyrimidin-4-ylamino)-benzenesulfonyl]-piperazin-1-yl}-ethanol (Ex. 88),
N -(N aphthalen-2-ylcarbamoylmethyl)-2-(2-pyridin-2-yl-6-pyridin-3-yl-pyrimidin-4-ylamino)-acetamide (Ex. 89),
{3-[4-(5-Methyl-furan-2-yl)-thiazol-2-yl]-phenyl}-(2-pyridin-2-yl-6-pyridin-3-yl-pyrimidin-4-yl)-amine (Ex. 90),
[2-(3-Methoxy-phenyl)-benzooxazol-5-yl]-(6-pyridin-3-yl-2-pyridin-2-yl-pyrimidin-4-yl)-amine (Ex. 91),
7-Methyl-2-[4-(2-pyridin-2-yl-6-pyridin-3-yl-pyrimidin-4-ylamino)-phenyl]-1H-benzoimidazol-5-ylamine (Ex. 92),
N-(1,1-Dioxo-1H-1lambda*6*-benzo[d]isothiazol-3-yl)-N'-(2-pyridin-2-yl-6-pyridin-3-yl-pyrimidin-4-yl)-ethane-1,2-diamine (Ex. 93),
{5-[2-Chloro-5-(6-pyridin-3-yl-2-pyridin-2-yl-pyrimidin-4-ylamino)-phenyl]-furan-2-yl]-methanol (Ex. 94),
1-Acetyl-3-[2-(6-pyridin-3-yl-2-pyridin-2-yl-pyrimidin-4-ylamino)-ethyl]-1H-indol-5-ol anion (Ex. 95),
1-(Naphthalen-2-yloxy)-3-(6-pyridin-3-yl-2-pyridin-2-yl-pyrimidin-4-ylamino)-propan-2-ol (Ex. 96),
4-(6-Pyridin-3-yl-2-pyridm-2-yl-pyrimidm-4-ylammo)-benzoic acid 2,3-dihydroxy-propyl ester (Ex. 97),
[1-Methyl-5-(6-pyridin-3-yl-2-pyridin-2-yl-pyrimidin-4-ylamino)-1H-benzoimidazol-2-yl]-methanol (Ex. 98),
(2-Pyridin-2-yl-6-pyridin-3-yl-pyrimidin-4-yl)-(3-thiophen-3-yl-phenyl)-amine (Ex. 99),
1-{5-Methoxy-3-[2-(6-pyridin-3-yl-2-pyridin-2-yl-pyrimidin-4-ylamino)-ethyl]-indol-1-yl}-ethanone (Ex. 100),
(3-Pyridin-4-yl-phenyl)-(2-pyridin-2-yl-6-pyridin-3-yl-pyrimidin-4-yl)-amine (Ex. 101),
(1S,2R)-1-Phenyl-2-(6-phenyl-2-pyridin-2-yl-pyrimidin-4-ylamino)-propane-1,3 diol (Ex. 102),
4-Bromo-N -[2-(6-pyridin-3-yl-2-pyridin-2-yl-pyrimidin-4-ylamino)-ethyl]-benzenesulfonamide (Ex. 103),
(1R,2R)-1-Phenyl-2-(2-pyridin-2-yl-6-pyridin-3-yl-pyrimidin-4-ylamino)-propan-1-ol (Ex. 104),
(S)-2-Amino-6-(6-pyridin-3-yl-2-pyridin-2-yl-pyrimidin-4-ylamino)-hexanoic acid amide (Ex. 105),
(3-Aminomethyl-cyclohexylmethyl)-(6-pyridin-3-yl-2-pyridin-2-yl-pyrimidin-4-yl)-amine (Ex. 106),
N-[4-(2-Pyridin-2-yl-6-pyridin-3-yl-pyrimidin-4-ylamino)-butyl]-guanidine (Ex. 107),
2-Ethyl-2-(2-pyridin-2-yl-6-pyridin-3-yl-pyrimidin-4-ylamino)-propane-1,3-diol (Ex. 108),
(1R,2R)-N-(2-Pyridin-2-y l-6-pyridin-3-yl-pyrimidin-4-yl)-cyclohexane-l,2-diamine (Ex. 109),
4-[2-(6-Pyridin-3-yl-2-pyridin-2-yl-pyrimidin-4-ylamino)-ethyl]-benzenesulfonamide (Ex. 110),
N*1*-(2-Pyridin-2-yl-6-pyridin-3-yl-pyrimidin-4-yl)-ethane-1,2-diamine (Ex. 111),
(4-Piperazin-1-yl-butyl)-(6-pyridin-3-yl-2-pyridin-2-yl-pyrimidin-4-yl)-amine (Ex. 112),
(6-Pyridin-3-yl-2-pyridin-2-yl-pyrimidin-4-yl)-(S)-1-pyrrolidin-2-ylmethyl-amine (Ex. 113),
[2-(4-Methyl-piperazin-1-yl)-ethyl]-(6-pyridin-3-yl-2-pyridin-2-yl-pyrimidin-4-yl)-amine (Ex. 114),
(1H-Imidazol-2-ylmethyl)-(6-pyridin-3-yl-2-pyridin-2-yl-pyrimidin-4-yl)-amine (Ex. 115),
3-(5-Methoxy-1H -indol-3-yl)-3-(6-pyridin-3-yl-2-pyridin-2-yl-pyrimidin-4-ylamino)-propan-1-ol (Ex. 116),
(2-Morpholin-2-yl-ethyl)-(6-pyridin-3-yl-2-pyridin-2-yl-pyrimidin-4-yl)-amine (Ex. 117),
[2-(7-Fluoro-1H-indol-3-yl)-ethyl]-(6-pyridin-3-yl-2-pyridin-2-yl-pyrimidin-4-yl)-amine (Ex. 118),
(5-Methyl-3-phenyl-isoxazol-4-ylmethyl)-(6-pyridin-3-yl-2-pyridin-2-yl-pyrimidin-4-yl)-amine (Ex. 119),
(5-Methyl-isoxazol-3-ylmethyl)-(6-pyridin-3-yl-2-pyridin-2-yl-pyrimidin-4-yl)-amine (Ex. 120),
(2-Morpholin-4-yl-pyridin-4-ylmethyl)-(6-pyridin-3-yl-2-pyridin-2-yl-pyrimidin-4-yl)-amine (Ex. 121),
(6-Piperazin-1-yl-pyridin-3-ylmethyl)-(6-pyridin-3-yl-2-pyridin-2-yl-pyrimidin-4-yl)-amine (Ex. 122),
4-[2-(6-Pyridin-3-yl-2-pyridin-2-yl-pyrimidin-4-ylamino)-ethyl]-phenol (Ex. 123),
Indan-1-yl-(6-pyridin-3-yl-2-pyridin-2-yl-pyrimidin-4-yl)-amine (Ex. 124),
Pyridin-2-ylmethyl-(6-pyridin-3-yl-2-pyridin-2-yl-pyrimidin-4-yl)-amine (Ex. 125),
Pyridin-3-ylmethyl-(6-pyridin-3-yl-2-pyridin-2-yl-pyrimidin-4-yl)-amine (Ex. 126),
Pyridin-4-ylmethyl-(6-pyridin-3-yl-2-pyridin-2-yl-pyrimidin-4-yl)-amine (Ex. 127),
5-[(6-Pyridin-3-yl-2-pyridin-2-yl-pyrimidin-4-ylamino)-methyl]-isoxazol-3-0l (Ex. 128),
(6-Pyridin-3-yl-2-pyridin-2-yl-pyrimidin-4-yl)-(1H-tetrazol-5-lmethyl)-amine (Ex. 129),
(5-Cyclopropyl-1H -pyrazol-4-ylmethyl)-(6-pyridin-3-yl-2-pyridin-2-yl-pyrimidin-4-yl)-amine (Ex. 130),
[2-(4-Methanesulfonyl-phenyl)-ethyl]-(6-pyridin-3-yl-2-pyridin-2-yl-pyrimidin-4-yl)-amine (Ex. 131),
[2-(4-Chloro-benzenesulfonyl)-ethyl]-(6-pyridin-3-yl-2-pyridin-2-yl-pyrimidin-4-yl)-amine (Ex. 132),
N*1*,N* 1*-Dimethyl-1-pyridin-3-yl-N*2*-(6-pyridin-3-yl-2-pyridin-2-yl-pyrimidin-4-yl)-ethane-1,2-diamine (Ex. 133),
(2-Morpholin-4-yl-2-pyridin-3-yl-ethyl)-(6-pyridin-3-yl-2-pyridin-2-yl-pyrimidin-4-yl)-amine (Ex. 134),
1-Pyridin-3-yl-2-(6-pyridin-3-yl-2-pyridin-2-yl-pyrimidin-4-ylamino)-ethanol (Ex. 135),
1-Pyridin-2-yl-2-(6-pyridin-3-yl-2-pyridin-2-yl-pyrimidin-4-ylamino)-ethanol (Ex. 136),
[2-(6-Methyl-pyridin-2-yl)-ethyl]-(6-pyridin-3-yl-2-pyridin-2-yl-pyrimidin-4-yl)-amine (Ex. 137),
1-Pyridin-4-yl-2-(6-pyridin-3-yl-2-pyridin-2-yl-pyrimidin-4-ylamino)-ethanol (Ex. 138),
[2-Morpholin-4-yl-2-(4-trifluoromethyl-phenyl)-ethyl]-(6-pyridin-3-yl-2-pyridin-2-yl-pyrimidin-4-yl)-amine (Ex. 139),
2-(1H-Indol-3-yl)-ethyl]-[2-(6-methyl-pyridin-2-yl)-6-pyridin-3-yl-pyrimidin-4-yl]-amine (Ex. 140),
[2-(1H-Indol-3-yl)-ethyl]-[2-(5-methyl-pyridin-2-yl)-6-pyridin-3-yl-pyrimidin-4-yl]-amine (Ex. 141),
[2-(1H-Indol-3-yl)-ethyl]-[2-(4-methyl-pyridin-2-yl)-6-pyridin-3-yl-pyrimidin-4-yl]-amine (Ex. 142),
[2-(1H-Indol-3-yl)-ethyl]-[2-(3-methyl-pyridin-2-yl)-6-pyridin-3-yl-pyrimidin-4-yl]-amine (Ex. 143),
are prepared by an analogous method to [2-(1H-indol-3-yl)-ethyl]-(6-pyridin-3-yl-2-pyridin-2-yl-pyrimidin-4-yl)-amine (Ex. 1).

### Example 144

### 6-Pyridin-3-yl-2-pyridin-2-yl-pyrimidin-4-ylamine

To 4-chloro-6-pyridin-3-yl-2-pyridin-2-yl-pyrimidine (intermediate 1b) (1eq, 1.1 mmol, 0.3 g) under inert atmosphere at 0°C is added a saturated solution of NH₃ (g) in MeOH (15 ml) in a sealed vessel. Reaction mixture is stirred at 140°C for 24 hours. Reaction mixture is cooled to room temperature and concentrated, extracted with hot chloroform, and the solvent evaporated under reduced pressure. The crude product is purified by flash chromatography (DCM) to afford titled compound as a white solid.

### Example 145

### 3-Benzooxazol-2-yl-N -(6-pyridin-3-yl-2-pyridin-2-yl-pyrimidin-4-yl)-propionamide

To a solution of 3-Benzooxazol-2-yl-propionic acid (1eq, 0.52 mmol, 0.1 g) in DMF (4 ml) is added a solution of DIPEA (2eq, 1.05 mmol, 0.18 ml) in DMF (4 ml), followed by a solution of HATU (1.2eq, 0.63 mmol, 0.24 g) in DMF (4 ml). Reaction mixture is stirred at room temperature for 10 minutes, to which a solution of 6-Pyridin-3-yl-2-pyridin-2-yl-pyrimidin-4-ylamine (Ex. 144) (1.2eq, 0.63 mmol, 0.16 g) in DMF (4 ml) is added. Reaction mixture is stirred at room temperature for 3 hours. Upon completion of reaction the solvent is removed under reduced pressure, and the residue is partitioned between DCM and 10% aqueous K₂CO₃. The combined organics were washed with brine, dried over MgSO₄ and filtered. The solvent is removed under reduced pressure and the crude product is purified by flash chromatography (DCM) to afford the titled compound.

### Examples 146 to 162

These examples namely,
3-(1H-Imidazol-2-yl)-N -(6-pyridin-3-yl-2-pyridin-2-yl-pyrimidin-4-yl)-propionamide (Ex. 146),
2-(1H-Benzoimidazol-2-yl)-N-(6-pyridin-3-yl-2-pyridin-2-yl-pyrimidin-4-yl)-acetamide (Ex. 147),
3-(1H-Benzoimidazol-2-yl)-N-(6-pyridin-3-yl-2-pyridin-2-yl-p yrimidin-4-yl)-propionamide (Ex. 148),
2-Benzooxazol-2-yl-N-(6-pyridin-3-yl-2-pyridin-2-yl-pyrimidin-4-yl)-acetamide (Ex. 149),
3-(3,5-Dimethyl-isoxazol-4-yl)-N-(6-pyridin-3-yl-2-pyridin-2-yl-pyrimidin-4-yl)-propionamide (Ex. 150),
3-(1H-Imidazol-4-yl)-N-(6-pyridin-3-yl-2-pyridin-2-yl-pyrimidin-4-yl)-propionamide (Ex. 151),
2-(3-Methyl-isoxazol-5-yl)-N-(6-pyridin-3-yl-2-pyridin-2-yl-pyrimidin-4-yl)-acetamide (Ex. 152),
3-(2,4-Dimethyl-1H-pyrrol-3-yl)-N-(6-pyridin-3-yl-2-pyridin-2-yl-pyrimidin-4-yl)-propionamide (Ex. 153),
3-(3-Pyrazin-2-yl-[1,2,4]oxadiazol-5-yl)-N-(6-pyridin-3-yl-2-pyridin-2-yl-pyrimidin-4-yl)-propionamide (Ex. 154),
2-(2-Methyl-1H-indol-3-yl)-N-(6-pyridin-3-yl-2-pyridin-2-yl-pyrimidin-4-yl)-acetamide (Ex. 155),
2-Benzo[d]isoxazol-3-yl-N-(6-pyridin-3-yl-2-pyridin-2-yl-pyrimidin-4-yl)-acetamide (Ex. 156),
3-(3-Pyridin-2-yl-[1,2,4]oxadiazol-5-yl)-N-(6-pyridin-3-yl-2-pyridin-2-yl-pyrimidin-4-yl)-propionamide (Ex. 157),
3-(1H-Indol-3-yl)-N-(6-pyridin-3-yl-2-pyridin-2-yl-pyrimidin-4-yl)-propionamide (Ex. 158),
2-(1H-Indol-3-yl)-N-(6-pyridin-3-yl-2-pyridin-2-yl-pyrimidin-4-yl)-acetamide (Ex. 159),
3-Pyridin-2-yl-N-(6-pyridin-3-yl-2-pyridin-2-yl-pyrimidin-4-yl)-propionamide (Ex. 160),
3-Benzo[1,3]dioxol-5-yl-N-(6-pyridin-3-yl-2-pyridin-2-yl-pyrimidin-4-yl)-propionamide (Ex. 161),
3-(4-Isopropyl-piperazin-1-yl)-N-(6-pyridin-3-yl-2-pyridin-2-yl-pyrimidin-4-yl)-propionamide (Ex. 162),
are prepared by an analogous method to 3-benzooxazol-2-yl-N-(6-pyridin-3-yl-2-pyridin-2-yl-pyrimidin-4-yl)-propionamide (Ex. 145).

### Example 163

### {6-[5-(4-Fluoro-phenyl)-pyridin-3-yl]-2-pyridin-2-yl-pyrimidin-4-yl}-[2-(1H-indol-3-yl)-ethyl]-amine

To a solution of [6-(5-Bromo-pyridin-3-yl)-2-pyridin-2-yl-pyrimidin-4-yl]-[2-(1H-indol-3-yl)-ethyl]-amine (1eq, 0.53 mmol, 0.25 g) in DME (6ml) under inert atmosphere is added 4-fluorophenyl boronic acid (1.2eq, 0.64 mmol, 0.09 g), Na₂CO₃ (2.3eq, 1.22 mmol, 0.13 g) in H₂O (2 ml), and tetrakis-(triphenylphoshine)palladium(0) (4%mol). Reaction mixture is heated at reflux for 3 hours. Upon consumption of starting material, the reaction mixture is cooled to room temperature, and H₂O is added. This aqueous is then extracted with EtOAc. The combined organics are washed with brine, dried over MgSO₄ and filtered. The solvent is removed under reduced pressure and the crude product purified by flash chromatography to afford the titled compound.

## Claims

1. A compound of formula I 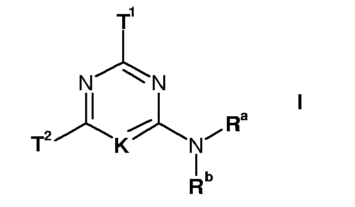 in free or salt or solvate form, where
T¹ is a 4- to 14-membered heterocyclic group optionally substituted at one, two or three positions by R¹, C₁-C₈-alkoxy, C₁-C₈-alkylthio, halo, halo-C₁-C₈-alkyl, amino, C₁-C₈-alkylamino, di(C₁-C₈-alkyl)amino, cyano, oxo, hydroxy, carboxy, nitro or by C₃-C₁₀-cycloalkyl optionally substituted at one, two or three positions by hydroxy, cyano, amino or R¹;
T² is a 4- to 14-membered heterocyclic group optionally substituted at one, two or three positions by R¹, R², R⁵, C₁-C₈-alkoxy, C₁-C₈-alkylthio, halo, halo-C₁-C₈-alkyl, amino, -C₁-C₈-alkylamino, di(C₁-C₈-alkyl)amino, cyano, oxo, hydroxy, carboxy, nitro or by C₃-C₁₀-cycloalkyl optionally substituted at one, two or three positions by hydroxy, cyano, amino or R¹;
K is -CR³ or N ;
either R^{a} and R^{b} are independently hydrogen;
C₁-C₈-alkyl optionally substituted at one, two or three positions by R⁴;
C₃-C₁₀-cycloalkyl optionally substituted at one, two or three positions by hydroxy, amino, C₁-C₈-alkoxy, C₁-C₈-alkylthio, halo, halo-C₁-C₈-alkyl, C₁-C₈-alkylamino, di(C₁-C₈-alkyl)amino, cyano, oxo, carboxy, nitro, -R¹, R², R⁵, -C(=O)-C₁-C₈-alkyl-R⁵, -C(=O)-C₁-C₈-alkyl-R², -C(=O)-NR⁶R⁷, -SO₂NR⁶R⁷, -SO₂-R² or -SO₂-R⁵, where C₃-C₁₀-cycloalkyl is optionally fused to a phenyl group;
-C(=O)-C₁-C₈-alkyl-R⁵, -C(=O)-R⁵, -C(=O)-C₁-C₈-alkyl-R², -C(=O)- R², -C(=O)-NR⁶R⁷, -SO₂NR⁶R⁷, -SO₂-R² or SO₂-R⁵;
a 4- to 14-membered heterocyclic group optionally substituted at one, two or three positions by hydroxy, C₁-C₈-alkoxy, C₁-C₈-alkylthio, halo, halo-C₁-C₈-alkyl, amino, C₁-C₈-alkylamino, di(C₁-C₈-alkyl)amino, cyano, oxo, carboxy, nitro, -R¹, -R², -R⁵ or -C(=O)-O-R¹; or
C₆-C₁₅-aryl optionally substituted at one, two or three positions by halo, hydroxy, C₁-C₈-alkoxy, C₁-C₈-alkylthio, halo-C₁-C₈-alkyl, amino, C₁-C₈-alkylamino, di(Ci-C₈-alkyl)amino, cyano, oxo, carboxy, nitro, -R⁵, -SO₂-R⁵ or -C(=O)-O-R¹;
or R^{a} and R^{b} together form a 4- to 14-membered heterocyclic group optionally substituted at one, two or three positions by oxo, -R¹, C₁-C₈-alkoxy, C₁-C₈-alkylthio, halo, amino, C₁-C₈-alkylamino, di(C₁-C₈-alkyl)amino, cyano, hydroxy, carboxy, -SO₂-R¹, -SO₂-R², -SO₂-R⁵, -SO₂NR⁶R⁷ or nitro;
R¹ is C₁-C₈-alkyl, C₂-C₈-alkenyl or C₂-C₈-alkynyl optionally substituted at one, two or three positions by hydroxy, cyano, amino, halo or -O-C₁-C₈-alkyl;
R² is C₆-C₁₅-aryl optionally substituted at one, two or three positions by halo, hydroxy, -R¹, -R⁵, C₁-C₈-alkoxy, C₁-C₈-alkylthio, amino, C₁-C₈-alkylamino, di(C₁-C₈-alkyl)amino, cyano, carboxy, nitro, -O-C₆-C₁₅-aryl, halo-C₁-C₈-alkyl, -NR⁶R⁷, -C₁-C₈-alkyl-NR⁶R⁷, -O-C₁-C₈-alkyl-NR⁶R⁷, -C₁-C₈-alkyl-R⁵, -O-R¹, -O-R⁵, -C(=O)-NH₂, -C(=O)NR⁶R⁷, -C(=O)-O-R¹, -O-C(=O)-R¹, -SO₂-NH₂, -SO₂-R¹, -SO₂-C₆-C₁₅-aryl, -SO₂-R⁵ or -SO₂NR⁶R⁷;
R³ is hydrogen, halo, cyano, amino or -R¹;
R⁴ is hydroxy, amino, C₁-C₈-alkoxy, C₁-C₈-alkylthio, halo, halo-C₁-C₈-alkyl, C₁-C₈-alkylamino, di(C₁-C₈-alkyl)amino, cyano, carboxy, nitro, -N(H)-C(=NH)-NH₂, -N(H)-SO₂-R², -R², -C(=O)-R², -C(=O)-R⁵, -O-R², -O-R⁵, -N(H)-R⁵, -N(H)-R², -NR⁶R⁷, -C(=O)-R¹, -C(=O)-NH₂, -SO₂-R⁵, -C(=O)-O-R¹, -C(=O)-O-R², -C(=O)-O-R⁵, -SO₂-R² or -C(=O)-N(H)-C₁-C₈-alkyl-C(=O)-N(H)-R²;
or R⁴ is a 4- to 14-membered heterocyclic group, that group being optionally substituted at one, two or three positions by hydroxy, halo, oxo, cyano, -R¹, amino, C₁-C₈-alkylamino, di(C₁-C₈-alkyl)amino, carboxy, nitro, -N(H)R¹, -C(=O)-NH₂, -C(=O)-NR⁶R⁷, -C(=O)-R², -C(=O)-R⁵ , C₁-C₈-alkoxy, C₁-C₈-alkylthio, C₃-C₁₀-cycloalkyl, -C(=O)-R¹, halo-C₁-C₈-alkyl, -R², -C₁-C₈-alkyl-R², -R⁵, -SO₂-R¹, -SO₂-R^{2,}, -SO₂-R² or -SO₂NR⁶R⁷;
or R⁴ is C₃-C₁₀-cycloalkyl substituted at one, two or three positions by hydroxy, amino, halo, C₁-C₈-alkylamino, di(C₁-C₈-alkyl)amino, cyano, carboxy, nitro -R¹, halo-C₁-C₈-alkyl, R², R⁵, -C(=O)-C₁-C₈-alkyl-R⁵, -C(=O)-C₁-C₈-alkyl-R², -C(=O)-NR⁶R⁷, -SO₂NR⁶R⁷, -SO₂-R², -SO₂-R⁵,
-C(=O)-NH₂, -SO₂-NH₂ or -SO₂-R¹;
R⁵ is a 4- to 14-membered heterocyclic group optionally substituted at one, two or three positions by oxo, amino, halo, C₁-C₈-alkylamino, di(C₁-C₈-alkyl)amino, cyano, hydroxy, carboxy, nitro, -R¹, C₁-C₈-alkoxy, C₁-C₈-alkylthio, halo-C₁-C₈-alkyl, -C(=O)-NH₂, -SO₂-NH₂,
-SO₂-C₆-C₁₅-aryl or by a 4- to 14-membered heterocyclic group optionally substituted at one, two or three positions by -R¹; and
R⁶ and R⁷ are independently hydrogen, -R¹, C₁-C₈-alkylamino, di(C₁-C₈-alkyl)amino, C₆-C₁₅-aryl, -C₁-C₈-alkyl-C₆-C₁₅-aryl, -R⁵ or -C₁-C₈-alkyl-R⁵.

2. A compound according to claim 1, wherein
T¹ is a 4- to 14-membered heterocyclic group optionally substituted at one, two or three positions by R¹;
T² is a 4- to 14-membered heterocyclic group optionally substituted at one, two or three positions by R²;
K is -CR³;
either R^{a} and R^{b} are independently hydrogen;
C₁-C₈-alkyl optionally substituted at one, two or three positions by R⁴,
C₃-C₁₀-cycloalkyl optionally substituted at one, two or three positions by hydroxy, amino, -R¹ or R², where C₃-C₁₀-cycloalkyl is optionally fused to a phenyl group; -C(=O)-C₁-C₈-alkyl-R⁵;
a 4- to 14-membered heterocyclic group optionally substituted at one, two or three positions by hydroxy, -R¹, -C(=O)-O-R¹ or -R²; or
C₆-C₁₅-aryl optionally substituted at one, two or three positions by halo, hydroxy, -R⁵, -SO₂-R⁵ or -C(=O)-O-R¹;
or R^{a} and R^{b} together form a 4- to 14-membered heterocyclic group optionally substituted at one, two or three positions by oxo;
R¹ is C₁-C₈-alkyl optionally substituted at one, two or three positions by hydroxy;
R² is C₆-C₁₅-aryl optionally substituted at one, two or three positions by halo, hydroxy, -R¹, C₁-C₈-alkoxy, -O-C₆-C₁₅-aryl, halo-C₁-C₈-alkyl, -SO₂-amino or -SO₂-C₁-C₈-alkyl;
R³ is hydrogen;
R⁴ is hydroxy, amino, -N(H)-C(=NH)-NH₂, -N(H)-SO₂-R², di(C₁-C₈-alkyl)amino, -R², -C(=O)-R⁵, -O-R², -O-R⁵, -N(H)-R⁵, -C(=O)-NH₂, -SO₂-R⁵, -C(=O)-O-R¹, -SO₂-R² or -C(=O)-N(H)-C₁-C₈-alkyl-C(=O)-N(H)-R² ;
or R⁴ is a 4- to 14-membered heterocyclic group, that group being optionally substituted at one, two or three positions by hydroxy, halo, oxo, cyano, -R¹, C₁-C₈-alkoxy, C₃-C₁₀-cycloalkyl, -C(=O)-R¹, halo-C₁-C₈-alkyl, -R², -C₁-C₈-alkyl-R² or -R⁵;
or R⁴ is C₃-C₁₀-cycloalkyl substituted at one, two or three positions by hydroxy, amino, -R¹, halo-C₁-C₈-alkyl, -SO₂-NH₂ or -SO₂-R¹; and
R⁵ is a 4- to 14-membered heterocyclic group optionally substituted at one, two or three positions by oxo, amino, halo, -R¹, C₁-C₈-alkoxy, halo-C₁-C₈-alkyl, -SO₂-C₆-C₁₅-aryl or by a 4- to 14-membered heterocyclic group optionally substituted at one, two or three positions by -R¹.

3. A compound according to claim 2, wherein
T¹ is a 4- to 14-membered heterocyclic group optionally substituted at one, two or three positions by R¹;
T² is a 4- to 14-membered heterocyclic group optionally substituted at one, two or three positions by R²;
K is -CR³ ;
either R^{a} and R^{b} are independently hydrogen;
C₁-C₅-alkyl optionally substituted at one, two or three positions by R⁴,
C₃-C₆-cycloalkyl optionally substituted at one, two or three positions by hydroxy, amino, -R¹ or R², where C₃-C₆-cycloalkyl is optionally fused to a phenyl group; -C(=O)-C₁-C₄-alkyl-R⁵;
a 4- to 10-membered heterocyclic group optionally substituted at one, two or three positions by hydroxy, -R¹, -C(=O)-O-R¹ or -R²; or
C₆-C₁₀-aryl optionally substituted at one, two or three positions by halo, hydroxy, -R⁵, -SO₂-R⁵ or -C(=O)-O-R¹;
or R^{a} and R^{b} together form a 4- to 10-membered heterocyclic group optionally substituted at one, two or three positions by oxo;
R¹ is C₁-C₄-alkyl optionally substituted at one, two or three positions by hydroxy;
R² is C₆-C₁₀-aryl optionally substituted at one, two or three positions by halo, hydroxy, -R¹, C₁-C₄-alkoxy, -O-C₆-C₁₀-aryl, halo-C₁-C₄-alkyl, -SO₂-amino or -SO₂-C₁-C₄-alkyl;
R³ is hydrogen;
R⁴ is hydroxy, amino, -N(H)-C(=NH)-NH₂, -N(H)-SO₂-R², di(C₁-C₄-alkyl)amino, -R², -C(=O)-R⁵, -O-R², -O-R⁵, -N(H)-R⁵, -C(=O)-NH₂, -SO₂-R⁵, -C(=O)-O-R¹, -SO₂-R² or -C(=O)-N(H)-C₁-C₄-alkyl-C(=O)-N(H)-R²;
or R⁴ is a 4- to 10-membered heterocyclic group, that group being optionally substituted at one, two or three positions by hydroxy, halo, oxo, cyano, -R¹, C₁-C₄-alkoxy, C₃-C₆-cycloalkyl, -C(=O)-R¹, halo-C₁-C₄-alkyl, -R², -C₁-C₄-alkyl-R², or -R⁵;
or R⁴ is C₃-C₆-cycloalkyl substituted at one, two or three positions by hydroxy, amino, -R¹, halo-C₁-C₄-alkyl, -SO₂-NH₂ or -SO₂-R¹; and
R⁵ is a 4- to 10-membered heterocyclic group optionally substituted at one, two or three positions by oxo, amino, halo, -R¹, C₁-C₄-alkoxy, halo-C₁-C₄-alkyl, -SO₂-C₆-C₁₀-aryl or by a 4- to 10-membered heterocyclic group optionally substituted at one, two or three positions by -R¹.

4. A compound according to claim 1 selected from [2-(1H-Indol-3-yl)-ethyl]-(6-pyridin-3-yl-2-pyridin-2-yl-pyrimidin-4-yl)-amine, [2-(5-Fluoro-1H-indol-3-yl)-ethyl]-(6-pyridin-3-yl-2-pyridin-2-yl-pyrimidin-4-yl)-amine, [2-(6-Fluoro-1H-indol-3-yl)-ethyl]-(6-pyridin-3-yl-2-pyridin-2-yl-pyrimidin-4-yl)-amine, (2-Pyridin-2-yl-ethyl)-(6-pyridin-3-yl-2-pyridin-2-yl-pyrimidin-4-yl)-amine, [2-(1H-Indol-3-yl)-ethyl]-(2-pyridin-3-yl-6-pyridin-2-yl-pyrimidin-4-yl)-amine, [2-(1-Methyl-1H-indol-3-yl)-ethyl]-(6-pyridin-3-yl-2-pyridin-2-yl-pyrimidin-4-yl)-amine, [2-(5-Chloro-1H-indol-3-yl)-ethyl]-(6-pyridin-3-yl-2-pyridin-2-yl-pyrimidin-4-yl)-amine and (2-Pyridin-3-yl-ethyl)-(6-pyridin-3-yl-2-pyridin-2-yl-pyrimidin-4-yl)-amine.

5. A compound according to claim 1 substantially as herein described with reference to any one of the Examples.

6. A compound according to any one of claims 1 to 4 in combination with another drug substance which is an anti-inflammatory, a bronchodilator, an antihistamine, a decongestant or an anti-tussive drug substance.

7. A compound according to any one of claims 1 to 4 for use as a pharmaceutical.

8. A pharmaceutical composition comprising as active ingredient a compound according to any one of claims 1 to 4 and a suitable pharmaceutically acceptable excipient.

9. The use of a compound according to any one of claims 1 to 4 for the manufacture of a medicament for the treatment of a condition mediated by the ALK-5 receptor.

10. The use of a compound according to any one of claims 1 to 4 for the manufacture of a medicament for the treatment of a condition mediated by the ALK-4 receptor.

11. The use of a compound according to any one of claims 1 to 4 for the manufacture of a medicament for the treatment of pulmonary hypertension, chronic renal disease, acute renal disease, wound healing, arthritis, osteoporosis, kidney disease, congestive heart failure, ulcers, ocular disorders, corneal wounds, diabetic nephropathy, impaired neuro-logical function, Alzheimer's disease, atherosclerosis, peritoneal and sub-dermal adhesion, kidney fibrosis, lung fibrosis and liver fibrosis, hepatitis B, hepatitis C, alcohol-induced hepatitis, haemochromatosis, primary biliary cirrhosis, restenosis, retroperitoneal fibrosis, mesenteric fibrosis, endometriosis, keloids, cancer, abnormal bone function, inflammatory disorders, scarring and photaging of the skin.

12. The use of a compound according to any one of claims 1 to 4 for the manufacture of a medicament for the treatment of pulmonary hypertension.

13. The use of a compound according to any one of claims 1 to 4 for the manufacture of a medicament for the treatment of pulmonary fibrosis.

14. The use of a compound according to any one of claims 1 to 4 for the manufacture of a medicament for the treatment of osteoporosis.

15. A process for the preparation of a compound of formula I as claimed in claim 1 which comprises reacting a compound of formula II 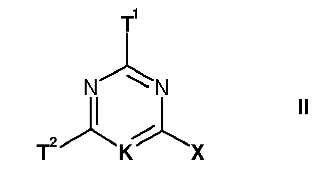 where T¹, T² and K are as defined in claim 1 and X is halo, with a compound of formula III 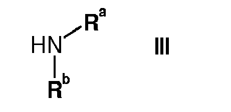 where R^{a} and R^{b} are as defined in claim 1.
